(19) Europäisches Patentamt
European Patent Office
Office européen des brevets

(11) EP 2 444 495 A1

(12) # EUROPEAN PATENT APPLICATION

(43) Date of publication:
**25.04.2012 Bulletin 2012/17**

(21) Application number: **10013808.0**

(22) Date of filing: **20.10.2010**

(51) Int Cl.:
***C12N 15/82*** *(2006.01)*
***C12P 21/02*** *(2006.01)*
*C07K 14/505* *(2006.01)*
*C07K 14/55* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Algenics**
**44800 Saint Herblain (FR)**

(72) Inventors:
- **Lejeune, Alexandre**
**44240 La Chapelle Sur Erdre (FR)**
- **Michel, Rémy**
**44100 Nantes (FR)**
- **Cadoret, Jean-Paul**
**44115 Basse Goulaine (FR)**
- **Carlier, Aude**
**44000 Nantes (FR)**

(74) Representative: **Barbot, Willy**
**SIMODORO**
**Parc Cézanne II, Bât. G**
**290 Avenue Galilée**
**13857 Aix en Provence Cedex 3 (FR)**

(54) **Secretion of recombinant polypeptides in the extracellular medium of diatoms**

(57) The present invention concerns a transformed diatom comprising a nucleic acid sequence operatively linked to a promoter, wherein said nucleic acid sequence encodes an amino acid sequence comprising (i) an heterologous signal peptide and (ii) a polypeptide, said heterologous signal peptide leading to the secretion of said polypeptide in the extracellular medium of said transformed diatom ; a method for producing a polypeptide which is secreted in the extracellular medium, said method comprising the steps of (i) culturing a transformed diatom, (ii) harvesting the extracellular medium of said culture and (iii) purifying the secreted polypeptide in said extracellular medium; and use of said transformed diatom for the secretion of a polypeptide in the extracellular medium.

EP 2 444 495 A1

SEQUENCE LISTING

<110> ALGENICS

<120> SECRETION OF RECOMBINANT POLYPEPTIDES IN THE EXTRACELLULAR MEDIUM OF DIATOMS

<130> ALG-B-0001 EP1

<160> 54

<170> PatentIn version 3.5

<210> 1
<211> 564
<212> DNA
<213> Homo sapiens

<400> 1
atgaccaaca agtgtctcct ccaaattgct ctcctgttgt gcttctccac tacagctctt 60

tccatgagct acaacttgct tggattccta caaagaagca gcaattttca gtgtcagaag 120

ctcctgtggc aattgaatgg gaggcttgaa tactgcctca aggacaggat gaactttgac 180

atccctgagg agattaagca gctgcagcag ttccagaagg aggacgccgc attgaccatc 240

tatgagatgc tccagaacat ctttgctatt ttcagacaag attcatctag cactggctgg 300

aatgagacta ttgttgagaa cctcctggct aatgtctatc atcagataaa ccatctgaag 360

acagtcctgg aagaaaaact ggagaaagaa gatttcacca ggggaaaact catgagcagt 420

ctgcacctga aaagatatta tgggaggatt ctgcattacc tgaaggccaa ggagtacagt 480

cactgtgcct ggaccatagt cagagtggaa atcctaagga acttttactt cattaacaga 540

cttacaggtt acctccgaaa ctga 564

<210> 2
<211> 567
<212> DNA
<213> Homo sapiens

<400> 2
atggccttga cctttgcttt actggtggcc ctcctggtgc tcagctgcaa gtcaagctgc 60

tctgtgggct gtgatctgcc tcaaacccac agcctgggta gcaggaggac cttgatgctc 120

ctggcacaga tgaggagaat ctctcttttc tcctgcttga aggacagaca tgactttgga 180

tttccccagg aggagtttgg caaccagttc caaaaggctg aaaccatccc tgtcctccat 240

gagatgatcc agcagatctt caatctcttc agcacaaagg actcatctgc tgcttgggat 300

gagaccctcc tagacaaatt ctacactgaa ctctaccagc agctgaatga cctggaagcc 360

tgtgtgatac agggggtggg ggtgacagag actcccctga tgaaggagga ctccattctg 420

gctgtgagga aatacttcca aagaatcact ctctatctga aagagaagaa atacagccct 480

tgtgcctggg aggttgtcag agcagaaatc atgagatctt tttctttgtc aacaaacttg 540

caagaaagtt taagaagtaa ggaatga 567

<210> 3
<211> 600
<212> DNA
<213> Homo sapiens

<400> 3
atgaactgtg tttgccgcct ggtcctggtc gtgctgagcc tgtggccaga tacagctgtc 60
gcccctgggc caccacctgg cccccctcga gtttccccag accctcgggc cgagctggac 120
agcaccgtgc tcctgacccg ctctctcctg gcggacacgc ggcagctggc tgcacagctg 180
agggacaaat tcccagctga cggggaccac aacctggatt ccctgcccac cctggccatg 240
agtgcggggg cactgggagc tctacagctc ccaggtgtgc tgacaaggct gcgagcggac 300
ctactgtcct acctgcggca cgtgcagtgg ctgcgccggg caggtggctc ttccctgaag 360
accctggagc ccgagctggg caccctgcag gcccgactgg accggctgct gcgccggctg 420
cagctcctga tgtcccgcct ggccctgccc cagccacccc cggacccgcc ggcgcccccg 480
ctggcgcccc cctcctcagc ctgggggggc atcagggccg cccacgccat cctggggggg 540
ctgcacctga cacttgactg ggccgtgagg ggactgctgc tgctgaagac tcggctgtga 600

<210> 4
<211> 462
<212> DNA
<213> Homo sapiens

<400> 4
atgtacagga tgcaactcct gtcttgcatt gcactaagtc ttgcacttgt cacaaacagt 60
gcacctactt caagttctac aaagaaaaca cagctacaac tggagcattt actgctggat 120
ttacagatga ttttgaatgg aattaataat tacaagaatc ccaaactcac caggatgctc 180
acatttaagt tttacatgcc caagaaggcc acagaactga aacatcttca gtgtctagaa 240
gaagaactca aacctctgga ggaagtgcta aatttagctc aaagcaaaaa ctttcactta 300
agacccaggg acttaatcag caatatcaac gtaatagttc tggaactaaa gggatctgaa 360
acaacattca tgtgtgaata tgctgatgag acagcaacca ttgtagaatt tctgaacaga 420
tggattacct tttgtcaaag catcatctca acactgactt ga 462

<210> 5
<211> 639
<212> DNA
<213> Homo sapiens

<400> 5
atgaactcct tctccacaag cgccttcggt ccagttgcct tctccctggg gctgctcctg 60
gtgttgcctg ctgccttccc tgccccagta cccccaggag aagattccaa agatgtagcc 120
gccccacaca gacagccact cacctcttca gaacgaattg acaaacaaat tcggtacatc 180
ctcgacggca tctcagccct gagaaaggag acatgtaaca agagtaacat gtgtgaaagc 240
agcaaagagg cactggcaga aaacaacctg aaccttccaa agatggctga aaaagatgga 300
tgcttccaat ctggattcaa tgaggagact tgcctggtga aaatcatcac tggtcttttg 360

gagtttgagg tatacctaga gtacctccag aacagatttg agagtagtga ggaacaagcc 420

agagctgtgc agatgagtac aaaagtcctg atccagttcc tgcagaaaaa ggcaaagaat 480

ctagatgcaa taaccacccc tgacccaacc acaaatgcca gcctgctgac gaagctgcag 540

gcacagaacc agtggctgca ggacatgaca actcatctca ttctgcgcag ctttaaggag 600

ttcctgcagt ccagcctgag ggctcttcgg caaatgtag 639

<210> 6
<211> 489
<212> DNA
<213> Homo sapiens

<400> 6
atgagatcca gtcctggcaa catggagagg attgtcatct gtctgatggt catcttcttg 60

gggacactgg tccacaaatc aagctcccaa ggtcaagatc gccacatgat tagaatgcgt 120

caacttatag atattgttga tcagctgaaa aattatgtga atgacttggt ccctgaattt 180

ctgccagctc cagaagatgt agagacaaac tgtgagtggt cagctttttc ctgctttcag 240

aaggcccaac taaagtcagc aaatacagga aacaatgaaa ggataatcaa tgtatcaatt 300

aaaaagctga agaggaaacc accttccaca aatgcaggga gaagacagaa acacagacta 360

acatgccctt catgtgattc ttatgagaaa aaaccaccca aagaattcct agaaagattc 420

aaatcacttc tccaaaagat gattcatcag catctgtcct ctagaacaca cggaagtgaa 480

gattcctga 489

<210> 7
<211> 333
<212> DNA
<213> Homo sapiens

<400> 7
atggccctgt ggatgcgcct cctgcccctg ctggcgctgc tggccctctg gggacctgac 60

ccagccgcag cctttgtgaa ccaacacctg tgcggctcac acctggtgga agctctctac 120

ctagtgtgcg gggaacgagg cttcttctac acacccaaga cccgccggga ggcagaggac 180

ctgcaggtgg ggcaggtgga gctgggcggg ggccctggtg caggcagcct gcagcccttg 240

gccctggagg ggtccctgca gaagcgtggc attgtggaac aatgctgtac cagcatctgc 300

tccctctacc agctggagaa ctactgcaac tag 333

<210> 8
<211> 540
<212> DNA
<213> Homo sapiens

<400> 8
atgaaaagca tttactttgt ggctggatta tttgtaatgc tggtacaagg cagctggcaa 60

cgttcccttc aagacacaga ggagaaatcc agatcattct cagcttccca ggcagaccca 120

ctcagtgatc ctgatcagat gaacgaggac aagcgccatt cacagggcac attcaccagt 180

gactacagca agtatctgga ctccaggcgt gcccaagatt ttgtgcagtg gttgatgaat 240

accaagagga acaggaataa cattgccaaa cgtcacgatg aatttgagag acatgctgaa 300
gggaccttta ccagtgatgt aagttcttat ttggaaggcc aagctgccaa ggaattcatt 360
gcttggctgg tgaaaggccg aggaaggcga gatttcccag aagaggtcgc cattgttgaa 420
gaacttggcc gcagacatgc tgatggttct ttctctgatg agatgaacac cattcttgat 480
aatcttgccg ccagggactt tataaactgg ttgattcaga ccaaaatcac tgacaggtga 540

<210> 9
<211> 582
<212> DNA
<213> Homo sapiens

<400> 9
atgggggtgc acgaatgtcc tgcctggctg tggcttctcc tgtccctgct gtcgctccct 60
ctgggcctcc cagtcctggg cgccccacca cgcctcatct gtgacagccg agtcctggag 120
aggtacctct tggaggccaa ggaggccgag aatatcacga cgggctgtgc tgaacactgc 180
agcttgaatg agaatatcac tgtcccagac accaaagtta atttctatgc ctggaagagg 240
atggaggtcg ggcagcaggc cgtagaagtc tggcagggcc tggccctgct gtcggaagct 300
gtcctgcggg gccaggccct gttggtcaac tcttcccagc cgtgggagcc cctgcagctg 360
catgtggata aagccgtcag tggccttcgc agcctcacca ctctgcttcg ggctctggga 420
gcccagaagg aagccatctc ccctccagat gcggcctcag ctgctccact ccgaacaatc 480
actgctgaca ctttccgcaa actcttccga gtctactcca atttcctccg gggaaagctg 540
aagctgtaca caggggaggc ctgcaggaca ggggacagat ga 582

<210> 10
<211> 654
<212> DNA
<213> Homo sapiens

<400> 10
atggctacag gctcccggac gtccctgctc ctggcttttg gcctgctctg cctgccctgg 60
cttcaagagg gcagtgcctt cccaaccatt cccttatcca ggctttttga caacgctatg 120
ctccgcgccc atcgtctgca ccagctggcc tttgacacct accaggagtt tgaagaagcc 180
tatatcccaa aggaacagaa gtattcattc ctgcagaacc cccagacctc cctctgtttc 240
tcagagtcta ttccgacacc ctccaacagg gaggaaacac aacagaaatc caacctagag 300
ctgctccgca tctccctgct gctcatccag tcgtggctgg agcccgtgca gttcctcagg 360
agtgtcttcg ccaacagcct ggtgtacggc gcctctgaca gcaacgtcta tgacctccta 420
aaggacctag aggaaggcat ccaaacgctg atggggaggc tggaagatgg cagcccccgg 480
actgggcaga tcttcaagca gacctacagc aagttcgaca caaactcaca caacgatgac 540
gcactactca agaactacgg gctgctctac tgcttcagga aggacatgga caaggtcgag 600
acattcctgc gcatcgtgca gtgccgctct gtggagggca gctgtggctt ctag 654

<210> 11
<211> 609
<212> DNA
<213> Homo sapiens

<400> 11
atggctacag gctcccggac gtccctgctc ctggcttttg gcctgctctg cctgccctgg 60

cttcaagagg gcagtgcctt cccaaccatt cccttatcca ggctttttga caacgctatg 120

ctccgcgccc atcgtctgca ccagctggcc tttgacacct accaggagtt taacccccag 180

acctccctct gtttctcaga gtctattccg acaccctcca acagggagga aacacaacag 240

aaatccaacc tagagctgct ccgcatctcc ctgctgctca tccagtcgtg gctggagccc 300

gtgcagttcc tcaggagtgt cttcgccaac agcctggtgt acggcgcctc tgacagcaac 360

gtctatgacc tcctaaagga cctagaggaa ggcatccaaa cgctgatggg gaggctggaa 420

gatggcagcc cccggactgg gcagatcttc aagcagacct acagcaagtt cgacacaaac 480

tcacacaacg atgacgcact actcaagaac tacgggctgc tctactgctt caggaaggac 540

atggacaagg tcgagacatt cctgcgcatc gtgcagtgcc gctctgtgga gggcagctgt 600

ggcttctag 609

<210> 12
<211> 534
<212> DNA
<213> Homo sapiens

<400> 12
atggctacag gctcccggac gtccctgctc ctggcttttg gcctgctctg cctgccctgg 60

cttcaagagg gcagtgcctt cccaaccatt cccttatcca ggctttttga caacgctatg 120

ctccgcgccc atcgtctgca ccagctggcc tttgacacct accaggagtt taacctagag 180

ctgctccgca tctccctgct gctcatccag tcgtggctgg agcccgtgca gttcctcagg 240

agtgtcttcg ccaacagcct ggtgtacggc gcctctgaca gcaacgtcta tgacctccta 300

aaggacctag aggaaggcat ccaaacgctg atggggaggc tggaagatgg cagcccccgg 360

actgggcaga tcttcaagca gacctacagc aagttcgaca caaactcaca caacgatgac 420

gcactactca agaactacgg gctgctctac tgcttcagga aggacatgga caaggtcgag 480

acattcctgc gcatcgtgca gtgccgctct gtggagggca gctgtggctt ctag 534

<210> 13
<211> 369
<212> DNA
<213> Homo sapiens

<400> 13
atggctacag gctcccggac gtccctgctc ctggcttttg gcctgctctg cctgccctgg 60

cttcaagagg gcagtgcctt cccaaccatt cccttatcca ggctttttga caacgctatg 120

ctccgcgccc atcgtctgca ccagctggcc tttgacacct accaggagtt taggctggaa 180

gatggcagcc cccggactgg gcagatcttc aagcagacct acagcaagtt cgacacaaac 240

tcacacaacg atgacgcact actcaagaac tacgggctgc tctactgctt caggaaggac 300

atggacaagg tcgagacatt cctgcgcatc gtgcagtgcc gctctgtgga gggcagctgt 360

ggcttctag 369

<210> 14
<211> 93
<212> DNA
<213> Homo sapiens

<400> 14
atggctacag aggctggaag atggcagccc ccggactggg cagatcttca agcagaccta 60

cagcaagttc gacacaaact cacacaacga tga 93

<210> 15
<211> 435
<212> DNA
<213> Homo sapiens

<400> 15
atgtggctgc agagcctgct gctcttgggc actgtggcct gcagcatctc tgcacccgcc 60

cgctcgccca gccccagcac gcagccctgg gagcatgtga atgccatcca ggaggcccgg 120

cgtctcctga acctgagtag agacactgct gctgagatga atgaaacagt agaagtcatc 180

tcagaaatgt ttgacctcca ggagccgacc tgcctacaga cccgcctgga gctgtacaag 240

cagggcctgc ggggcagcct caccaagctc aagggcccct tgaccatgat ggccagccac 300

tacaagcagc actgccctcc aaccccggaa acttcctgtg caacccagat tatcaccttt 360

gaaagtttca aagagaacct gaaggacttt ctgcttgtca tcccctttga ctgctgggag 420

ccagtccagg agtga 435

<210> 16
<211> 624
<212> DNA
<213> Homo sapiens

<400> 16
atggctggac ctgccaccca gagccccatg aagctgatgg ccctgcagct gctgctgtgg 60

cacagtgcac tctggacagt gcaggaagcc accccccctgg gccctgccag ctccctgccc 120

cagagcttcc tgctcaagtg cttagagcaa gtgaggaaga tccagggcga tggcgcagcg 180

ctccaggaga agctggtgag tgagtgtgcc acctacaagc tgtgccaccc cgaggagctg 240

gtgctgctcg gacactctct gggcatcccc tgggctcccc tgagcagctg ccccagccag 300

gccctgcagc tggcaggctg cttgagccaa ctccatagcg gccttttcct ctaccagggg 360

ctcctgcagg ccctggaagg gatctccccc gagttgggtc ccaccttgga cacactgcag 420

ctggacgtcg ccgactttgc caccaccatc tggcagcaga tggaagaact gggaatggcc 480

cctgccctgc agcccaccca gggtgccatg ccggccttcg cctctgcttt ccagcgccgg 540

gcaggagggg tcctggttgc ctcccatctg cagagcttcc tggaggtgtc gtaccgcgtt 600

ctacgccacc ttgcccagcc ctga 624

<210> 17
<211> 351
<212> DNA
<213> Homo sapiens

<400> 17
atggattact acagaaaata tgcagctatc tttctggtca cattgtcggt gtttctgcat 60

gttctccatt ccgctcctga tgtgcaggat tgcccagaat gcacgctaca ggaaaaccca 120

ttcttctccc agccgggtgc cccaatactt cagtgcatgg gctgctgctt ctctagagca 180

tatcccactc cactaaggtc caagaagacg atgttggtcc aaaagaacgt cacctcagag 240

tccacttgct gtgtagctaa atcatataac agggtcacag taatgggggg tttcaaagtg 300

gagaaccaca cggcgtgcca ctgcagtact tgttattatc acaaatctta a 351

<210> 18
<211> 390
<212> DNA
<213> Homo sapiens

<400> 18
atgaagacac tccagttttt cttccttttc tgttgctgga aagcaatctg ctgcaatagc 60

tgtgagctga ccaacatcac cattgcaata gagaaagaag aatgtcgttt ctgcataagc 120

atcaacacca cttggtgtgc tggctactgc tacaccaggg atctggtgta taaggaccca 180

gccaggccca aaatccagaa aacatgtacc ttcaaggaac tggtatacga aacagtgaga 240

gtgcccggct gtgctcacca tgcagattcc ttgtatacat acccagtggc cacccagtgt 300

cactgtggca agtgtgacag cgacagcact gattgtactg tgcgaggcct ggggcccagc 360

tactgctcct ttggtgaaat gaaagaataa 390

<210> 19
<211> 498
<212> DNA
<213> Homo sapiens

<400> 19
atggagatgt tccaggggct gctgctgttg ctgctgctga gcatgggcgg gacatgggca 60

tccaaggagc cgcttcggcc acggtgccgc cccatcaatg ccaccctggc tgtggagaag 120

gagggctgcc ccgtgtgcat caccgtcaac accaccatct gtgccggcta ctgccccacc 180

atgacccgcg tgctgcaggg ggtcctgccg gccctgcctc aggtggtgtg caactaccgc 240

gatgtgcgct tcgagtccat ccggctccct ggctgcccgc gcggcgtgaa ccccgtggtc 300

tcctacgccg tggctctcag ctgtcaatgt gcactctgcc gccgcagcac cactgactgc 360

gggggtccca aggaccaccc cttgacctgt gatgaccccc gcttccagga ctcctcttcc 420

tcaaaggccc ctcccccag ccttccaagc ccatcccgac tcccggggcc ctcggacacc 480

ccgatcctcc cacaataa 498

<210> 20

<211> 417
<212> DNA
<213> Homo sapiens

<400> 20
atgactgctc tctttctgat gtccatgctt tttggcctta catgtgggca agcgatgtct 60

ttttgtattc caactgagta tacaatgcac atcgaaagga gagagtgtgc ttattgccta 120

accatcaaca ccaccatctg tgctggatat tgtatgacac gggatatcaa tggcaaactg 180

tttcttccca aatatgctct gtcccaggat gtttgcacat atagagactt catctacagg 240

actgtagaaa taccaggatg cccactccat gttgctccct atttttccta tcctgttgct 300

ttaagctgta agtgtggcaa gtgcaatact gactatagtg actgcataca tgaagccatc 360

aagacaaact actgtaccaa acctcagaag tcttatctgg taggattttc tgtctaa 417

<210> 21
<211> 426
<212> DNA
<213> Homo sapiens

<400> 21
atggagatgc tccaggggct gctgctgttg ctgctgctga gcatgggcgg ggcatgggca 60

tccagggagc cgcttcggcc atggtgccac cccatcaatg ccatcctggc tgtcgagaag 120

gagggctgcc cagtgtgcat caccgtcaac accaccatct gtgccggcta ctgccccacc 180

atgatgcgcg tgctgcaggc ggtcctgccg cccctgcctc aggtggtgtg cacctaccgt 240

gatgtgcgct tcgagtccat ccggctccct ggctgcccgc gtggtgtgga ccccgtggtc 300

tccttccctg tggctctcag ctgtcgctgt ggaccctgcc gccgcagcac ctctgactgt 360

gggggtccca aagaccaccc cttgacctgt gaccaccccc aactctcagg cctcctcttc 420

ctctaa 426

<210> 22
<211> 1869
<212> DNA
<213> Homo sapiens

<400> 22
atggcgcacg tccgaggctt gcagctgcct ggctgcctgg ccctggctgc cctgtgtagc 60

cttgtgcaca gccagcatgt gttcctggct cctcagcaag cacggtcgct gctccagcgg 120

gtccggcgag ccaacacctt cttggaggag gtgcgcaagg gcaacctgga gcgagagtgc 180

gtggaggaga cgtgcagcta cgaggaggcc ttcgaggctc tggagtcctc cacggctacg 240

gatgtgttct gggccaagta cacagcttgt gagacagcga ggacgcctcg agataagctt 300

gctgcatgtc tggaaggtaa ctgtgctgag ggtctgggta cgaactaccg agggcatgtg 360

aacatcaccc ggtcaggcat tgagtgccag ctatggagga gtcgctaccc acataagcct 420

gaaatcaact ccactaccca tcctggggcc gacctacagg agaatttctg ccgcaacccc 480

gacagcagca ccacgggacc ctggtgctac actacagacc ccaccgtgag gaggcaggaa 540

tgcagcatcc ctgtctgtgg ccaggatcaa gtcactgtag cgatgactcc acgctccgaa 600

ggctccagtg tgaatctgtc acctccattg gagcagtgtg tccctgatcg ggggcagcag 660
taccaggggc gcctggcggt gaccacacat gggctcccct gcctggcctg ggccagcgca 720
caggccaagg ccctgagcaa gcaccaggac ttcaactcag ctgtgcagct ggtggagaac 780
ttctgccgca acccagacgg ggatgaggag ggcgtgtggt gctatgtggc cgggaagcct 840
ggcgactttg ggtactgcga cctcaactat tgtgaggagg ccgtggagga ggagacagga 900
gatgggctgg atgaggactc agacagggcc atcgaagggc gtaccgccac cagtgagtac 960
cagactttct tcaatccgag gacctttggc tcgggagagg cagactgtgg gctgcgacct 1020
ctgttcgaga agaagtcgct ggaggacaaa accgaaagag agctcctgga atcctacatc 1080
gacgggcgca ttgtggaggg ctcggatgca gagatcggca tgtcaccttg gcaggtgatg 1140
cttttccgga agagtcccca ggagctgctg tgtggggcca gcctcatcag tgaccgctgg 1200
gtcctcaccg ccgcccactg cctcctgtac ccgccctggg acaagaactt caccgagaat 1260
gaccttctgg tgcgcattgg caagcactcc cgcaccaggt acgagcgaaa cattgaaaag 1320
atatccatgt tggaaaagat ctacatccac cccaggtaca actggcggga gaacctggac 1380
cgggacattg ccctgatgaa gctgaagaag cctgttgcct tcagtgacta cattcaccct 1440
gtgtgtctgc ccgacaggga gacggcagcc agcttgctcc aggctggata caaggggcgg 1500
gtgacaggct ggggcaacct gaaggagacg tggacagcca acgttggtaa ggggcagccc 1560
agtgtcctgc aggtggtgaa cctgcccatt gtggagcggc cggtctgcaa ggactccacc 1620
cggatccgca tcactgacaa catgttctgt gctggttaca agcctgatga agggaaacga 1680
ggggatgcct gtgaaggtga cagtggggga ccctttgtca tgaagagccc ctttaacaac 1740
cgctggtatc aaatgggcat cgtctcatgg ggtgaaggct gtgaccggga tgggaaatat 1800
ggcttctaca cacatgtgtt ccgcctgaag aagtggatac agaaggtcat tgatcagttt 1860
ggagagtag 1869

<210> 23
<211> 1401
<212> DNA
<213> Homo sapiens

<400> 23
atggtctccc aggccctcag gctcctctgc cttctgcttg ggcttcaggg ctgcctggct 60
gcaggcgggg tcgctaaggc ctcaggagga gaaacacggg acatgccgtg gaagccgggg 120
cctcacagag tcttcgtaac ccaggaggaa gcccacggcg tcctgcaccg gcgccggcgc 180
gccaacgcgt tcctggagga gctgcggccg ggctccctgg agagggagtg caaggaggag 240
cagtgctcct tcgaggaggc ccgggagatc ttcaaggacg cggagaggac gaagctgttc 300
tggatttctt acagtgatgg ggaccagtgt gcctcaagtc catgccagaa tgggggctcc 360
tgcaaggacc agctccagtc ctatatctgc ttctgcctcc ctgccttcga gggccggaac 420
tgtgagacgc acaaggatga ccagctgatc tgtgtgaacg agaacggcgg ctgtgagcag 480

tactgcagtg accacacggg caccaagcgc tcctgtcggt gccacgaggg gtactctctg 540
ctggcagacg gggtgtcctg cacacccaca gttgaatatc catgtggaaa aatacctatt 600
ctagaaaaaa gaaatgccag caaaccccaa ggccgaattg tgggggggcaa ggtgtgcccc 660
aaaggggagt gtccatggca ggtcctgttg ttggtgaatg gagctcagtt gtgtgggggg 720
accctgatca acaccatctg ggtggtctcc gcggcccact gtttcgacaa aatcaagaac 780
tggaggaacc tgatcgcggt gctgggcgag cacgacctca gcgagcacga cggggatgag 840
cagagccggc gggtggcgca ggtcatcatc cccagcacgt acgtcccggg caccaccaac 900
cacgacatcg cgctgctccg cctgcaccag cccgtggtcc tcactgacca tgtggtgccc 960
ctctgcctgc ccgaacggac gttctctgag aggacgctgg ccttcgtgcg cttctcattg 1020
gtcagcggct ggggccagct gctggaccgt ggcgccacgg ccctggagct catggtcctc 1080
aacgtgcccc ggctgatgac ccaggactgc ctgcagcagt cacggaaggt gggagactcc 1140
ccaaatatca cggagtacat gttctgtgcc ggctactcgg atggcagcaa ggactcctgc 1200
aagggggaca gtggaggccc acatgccacc cactaccggg gcacgtggta cctgacgggc 1260
atcgtcagct ggggccaggg ctgcgcaacc gtgggccact ttggggtgta caccagggtc 1320
tcccagtaca tcgagtggct gcaaaagctc atgcgctcag agccacgccc aggagtcctc 1380
ctgcgagccc catttcccta g 1401

<210> 24
<211> 7056
<212> DNA
<213> Homo sapiens

<400> 24
atgcaaatag agctctccac ctgcttcttt ctgtgccttt tgcgattctg ctttagtgcc 60
accagaagat actacctggg tgcagtggaa ctgtcatggg actatatgca aagtgatctc 120
ggtgagctgc ctgtggacgc aagatttcct cctagagtgc caaaatcttt tccattcaac 180
acctcagtcg tgtacaaaaa gactctgttt gtagaattca cggttcacct tttcaacatc 240
gctaagccaa ggccaccctg gatgggtctg ctaggtccta ccatccaggc tgaggtttat 300
gatacagtgg tcattacact taagaacatg gcttcccatc ctgtcagtct tcatgctgtt 360
ggtgtatcct actggaaagc ttctgaggga gctgaatatg atgatcagac cagtcaaagg 420
gagaaagaag atgataaagt cttccctggt ggaagccata catatgtctg gcaggtcctg 480
aaagagaatg gtccaatggc ctctgaccca ctgtgcctta cctactcata tctttctcat 540
gtggacctgg taaaagactt gaattcaggc ctcattggag ccctactagt atgtagagaa 600
gggagtctgg ccaaggaaaa gacacagacc ttgcacaaat ttatactact tttgctgta 660
tttgatgaag ggaaaagttg gcactcagaa acaaagaact ccttgatgca ggatagggat 720
gctgcatctg ctcgggcctg gcctaaaatg cacacagtca atggttatgt aaacaggtct 780
ctgccaggtc tgattggatg ccacaggaaa tcagtctatt ggcatgtgat tggaatgggc 840
accactcctg aagtgcactc aatattcctc gaaggtcaca catttcttgt gaggaaccat 900

cgccaggcgt ccttggaaat ctcgccaata actttcctta ctgctcaaac actcttgatg 960
gaccttggac agtttctact gttttgtcat atctcttccc accaacatga tggcatggaa 1020
gcttatgtca aagtagacag ctgtccagag gaaccccaac tacgaatgaa aaataatgaa 1080
gaagcggaag actatgatga tgatcttact gattctgaaa tggatgtggt caggtttgat 1140
gatgacaact ctccttcctt tatccaaatt cgctcagttg ccaagaagca tcctaaaact 1200
tgggtacatt acattgctgc tgaagaggag gactgggact atgctccctt agtcctcgcc 1260
cccgatgaca gaagttataa aagtcaatat ttgaacaatg gccctcagcg gattggtagg 1320
aagtacaaaa aagtccgatt tatggcatac acagatgaaa cctttaagac tcgtgaagct 1380
attcagcatg aatcaggaat cttgggacct ttactttatg gggaagttgg agacacactg 1440
ttgattatat ttaagaatca agcaagcaga ccatataaca tctaccctca cggaatcact 1500
gatgtccgtc ctttgtattc aaggagatta ccaaaaggtg taaaacattt gaaggatttt 1560
ccaattctgc caggagaaat attcaaatat aaatggacag tgactgtaga agatgggcca 1620
actaaatcag atcctcggtg cctgacccgc tattactcta gtttcgttaa tatggagaga 1680
gatctagctt caggactcat tggccctctc ctcatctgct acaaagaatc tgtagatcaa 1740
agaggaaacc agataatgtc agacaagagg aatgtcatcc tgttttctgt atttgatgag 1800
aaccgaagct ggtacctcac agagaatata caacgctttc tccccaatcc agctggagtg 1860
cagcttgagg atccagagtt ccaagcctcc aacatcatgc acagcatcaa tggctatgtt 1920
tttgatagtt tgcagttgtc agtttgtttg catgaggtgg catactggta cattctaagc 1980
attggagcac agactgactt cctttctgtc ttcttctctg gatatacctt caaacacaaa 2040
atggtctatg aagacacact caccctattc ccattctcag gagaaactgt cttcatgtcg 2100
atggaaaacc caggtctatg gattctgggg tgccacaact cagactttcg gaacagaggc 2160
atgaccgcct tactgaaggt ttctagttgt gacaagaaca ctggtgatta ttacgaggac 2220
agttatgaag atatttcagc atacttgctg agtaaaaaca atgccattga accaagaagc 2280
ttctcccaga attcaagaca ccctagcact aggcaaaagc aatttaatgc caccacaatt 2340
ccagaaaatg acatagagaa gactgaccct tggtttgcac acagaacacc tatgcctaaa 2400
atacaaaatg tctcctctag tgatttgttg atgctcttgc gacagagtcc tactccacat 2460
gggctatcct tatctgatct ccaagaagcc aaatatgaga ctttttctga tgatccatca 2520
cctggagcaa tagacagtaa taacagcctg tctgaaatga cacacttcag gccacagctc 2580
catcacagtg gggacatggt atttacccct gagtcaggcc tccaattaag attaaatgag 2640
aaactgggga caactgcagc aacagagttg aagaaacttg atttcaaagt ttctagtaca 2700
tcaaataatc tgatttcaac aattccatca gacaatttgg cagcaggtac tgataataca 2760
agttccttag gacccccaag tatgccagtt cattatgata gtcaattaga taccactcta 2820
tttggcaaaa agtcatctcc ccttactgag tctggtggac ctctgagctt gagtgaagaa 2880
aataatgatt caaagttgtt agaatcaggt ttaatgaata gccaagaaag ttcatgggga 2940

aaaaatgtat cgtcaacaga gagtggtagg ttatttaaag ggaaaagagc tcatggacct 3000
gctttgttga ctaaagataa tgccttattc aaagttagca tctctttgtt aaagacaaac 3060
aaaacttcca ataattcagc aactaataga aagactcaca ttgatggccc atcattatta 3120
attgagaata gtccatcagt ctggcaaaat atattagaaa gtgacactga gtttaaaaaa 3180
gtgacacctt tgattcatga cagaatgctt atggacaaaa atgctacagc tttgaggcta 3240
aatcatatgt caaataaaac tacttcatca aaaaacatgg aaatggtcca acagaaaaaa 3300
gagggcccca ttccaccaga tgcacaaaat ccagatatgt cgttctttaa gatgctattc 3360
ttgccagaat cagcaaggtg gatacaaagg actcatggaa agaactctct gaactctggg 3420
caaggcccca gtccaaagca attagtatcc ttaggaccag aaaaatctgt ggaaggtcag 3480
aatttcttgt ctgagaaaaa caaagtggta gtaggaaagg gtgaatttac aaaggacgta 3540
ggactcaaag agatggtttt tccaagcagc agaaacctat ttcttactaa cttggataat 3600
ttacatgaaa ataatacaca caatcaagaa aaaaaaattc aggaagaaat agaaaagaag 3660
gaaacattaa tccaagagaa tgtagttttg cctcagatac atacagtgac tggcactaag 3720
aatttcatga agaacctttt cttactgagc actaggcaaa atgtagaagg ttcatatgag 3780
ggggcatatg ctccagtact tcaagatttt aggtcattaa atgattcaac aaatagaaca 3840
aagaaacaca cagctcattt ctcaaaaaaa ggggaggaag aaaacttgga aggcttggga 3900
aatcaaacca agcaaattgt agagaaatat gcatgcacca caaggatatc tcctaataca 3960
agccagcaga attttgtcac gcaacgtagt aagagagctt tgaaacaatt cagactccca 4020
ctagaagaaa cagaacttga aaaaaggata attgtggatg acacctcaac ccagtggtcc 4080
aaaaacatga aacatttgac cccgagcacc ctcacacaga tagactacaa tgagaaggag 4140
aaaggggcca ttactcagtc tcccttatca gattgcctta cgaggagtca tagcatccct 4200
caagcaaata gatctccatt acccattgca aaggtatcat catttccatc tattagacct 4260
atatatctga ccagggtcct attccaagac aactcttctc atcttccagc agcatcttat 4320
agaaagaaag attctggggt ccaagaaagc agtcatttct tacaaggagc caaaaaaaat 4380
aacctttctt tagccattct aaccttggag atgactggtg atcaaagaga ggttggctcc 4440
ctggggacaa gtgccacaaa ttcagtcaca tacaagaaag ttgagaacac tgttctcccg 4500
aaaccagact tgcccaaaac atctggcaaa gttgaattgc ttccaaaagt tcacatttat 4560
cagaaggacc tattccctac ggaaactagc aatgggtctc ctggccatct ggatctcgtg 4620
gaagggagcc ttcttcaggg aacagaggga gcgattaagt ggaatgaagc aaacagacct 4680
ggaaaagttc cctttctgag agtagcaaca gaaagctctg caaagactcc ctccaagcta 4740
ttggatcctc ttgcttggga taaccactat ggtactcaga taccaaaaga agagtggaaa 4800
tcccaagaga agtcaccaga aaaaacagct tttaagaaaa aggataccat tttgtccctg 4860
aacgcttgtg aaagcaatca tgcaatagca gcaataaatg agggacaaaa taagcccgaa 4920
atagaagtca cctgggcaaa gcaaggtagg actgaaaggc tgtgctctca aaacccacca 4980

gtcttgaaac gccatcaacg ggaaataact cgtactactc ttcagtcaga tcaagaggaa 5040
attgactatg atgataccat atcagttgaa atgaagaagg aagattttga catttatgat 5100
gaggatgaaa atcagagccc ccgcagcttt caaaagaaaa cacgacacta ttttattgct 5160
gcagtggaga ggctctggga ttatgggatg agtagctccc cacatgttct aagaaacagg 5220
gctcagagtg gcagtgtccc tcagttcaag aaagttgttt tccaggaatt tactgatggc 5280
tcctttactc agcccttata ccgtggagaa ctaaatgaac atttgggact cctggggcca 5340
tatataagag cagaagttga agataatatc atggtaactt tcagaaatca ggcctctcgt 5400
ccctattcct tctattctag ccttatttct tatgaggaag atcagaggca aggagcagaa 5460
cctagaaaaa actttgtcaa gcctaatgaa accaaaactt acttttggaa agtgcaacat 5520
catatggcac ccactaaaga tgagtttgac tgcaaagcct gggcttattt ctctgatgtt 5580
gacctggaaa aagatgtgca ctcaggcctg attggacccc ttctggtctg ccacactaac 5640
acactgaacc ctgctcatgg gagacaagtg acagtacagg aatttgctct gtttttcacc 5700
atctttgatg agaccaaaag ctggtacttc actgaaaata tggaaagaaa ctgcagggct 5760
ccctgcaata tccagatgga agatcccact tttaaagaga attatcgctt ccatgcaatc 5820
aatggctaca taatggatac actacctggc ttagtaatgg ctcaggatca aaggattcga 5880
tggtatctgc tcagcatggg cagcaatgaa aacatccatt ctattcattt cagtggacat 5940
gtgttcactg tacgaaaaaa agaggagtat aaaatggcac tgtacaatct ctatccaggt 6000
gtttttgaga cagtggaaat gttaccatcc aaagctggaa tttggcgggt ggaatgcctt 6060
attggcgagc atctacatgc tgggatgagc acactttttc tggtgtacag caataagtgt 6120
cagactcccc tgggaatggc ttctggacac attagagatt ttcagattac agcttcagga 6180
caatatggac agtgggcccc aaagctggcc agacttcatt attccggatc aatcaatgcc 6240
tggagcacca aggagccctt ttcttggatc aaggtggatc tgttggcacc aatgattatt 6300
cacggcatca agacccaggg tgcccgtcag aagttctcca gcctctacat ctctcagttt 6360
atcatcatgt atagtcttga tgggaagaag tggcagactt atcgaggaaa ttccactgga 6420
accttaatgg tcttctttgg caatgtggat tcatctggga taaaacacaa tatttttaac 6480
cctccaatta ttgctcgata catccgtttg cacccaactc attatagcat tcgcagcact 6540
cttcgcatgg agttgatggg ctgtgattta aatagttgca gcatgccatt gggaatggag 6600
agtaaagcaa tatcagatgc acagattact gcttcatcct actttaccaa tatgtttgcc 6660
acctggtctc cttcaaaagc tcgacttcac ctccaaggga ggagtaatgc ctggagacct 6720
caggtgaata atccaaaaga gtggctgcaa gtggacttcc agaagacaat gaaagtcaca 6780
ggagtaacta ctcagggagt aaaatctctg cttaccagca tgtatgtgaa ggagttcctc 6840
atctccagca gtcaagatgg ccatcagtgg actctctttt ttcagaatgg caaagtaaag 6900
gtttttcagg gaaatcaaga ctccttcaca cctgtggtga actctctaga cccaccgtta 6960
ctgactcgct accttcgaat tcacccccag agttgggtgc accagattgc cctgaggatg 7020

gaggttctgg gctgcgaggc acaggacctc tactga 7056

<210> 25
<211> 1389
<212> DNA
<213> Homo sapiens

<400> 25
atgcagcgcg tgaacatgat catggcagaa tcaccaagcc tcatcaccat ctgcctttta 60

ggatatctac tcagtgctga atgtacagtt tttcttgatc atgaaaacgc caacaaaatt 120

ctgaatcggc caaagaggta taattcaggt aaattggaag agtttgttca agggaacctt 180

gagagagaat gtatggaaga aaagtgtagt tttgaagaac cacgagaagt ttttgaaaac 240

actgaaaaga caactgaatt ttggaagcag tatgttgatg gagatcagtg tgagtccaat 300

ccatgtttaa atggcggcag ttgcaaggat gacattaatt cctatgaatg ttggtgtccc 360

tttggatttg aaggaaagaa ctgtgaatta gatgtaacat gtaacattaa gaatggcaga 420

tgcgagcagt tttgtaaaaa tagtgctgat aacaaggtgg tttgctcctg tactgaggga 480

tatcgacttg cagaaaacca gaagtcctgt gaaccagcag tgccatttcc atgtggaaga 540

gtttctgttt cacaaacttc taagctcacc cgtgctgagg ctgtttttcc tgatgtggac 600

tatgtaaatc ctactgaagc tgaaaccatt ttggataaca tcactcaagg cacccaatca 660

tttaatgact tcactcgggt tgttggtgga gaagatgcca aaccaggtca attcccttgg 720

caggttgttt tgaatggtaa agttgatgca ttctgtggag gctctatcgt taatgaaaaa 780

tggattgtaa ctgctgccca ctgtgttgaa actggtgtta aaattacagt tgtcgcaggt 840

gaacataata ttgaggagac agaacataca gagcaaaagc gaaatgtgat tcgagcaatt 900

attcctcacc acaactacaa tgcagctatt aataagtaca accatgacat tgcccttctg 960

gaactggacg aacccttagt gctaaacagc tacgttacac ctatttgcat tgctgacaag 1020

gaatacacga acatcttcct caaatttgga tctggctatg taagtggctg ggcaagagtc 1080

ttccacaaag ggagatcagc tttagttctt cagtacctta gagttccact tgttgaccga 1140

gccacatgtc ttcgatctac aaagttcacc atctataaca acatgttctg tgctggcttc 1200

catgaaggag gtagagattc atgtcaagga gatagtgggg gaccccatgt tactgaagtg 1260

gaagggacca gtttcttaac tggaattatt agctggggtg aagagtgtgc aatgaaaggc 1320

aaatatggaa tatataccaa ggtatcccgg tatgtcaact ggattaagga aaaaacaaag 1380

ctcacttaa 1389

<210> 26
<211> 1551
<212> DNA
<213> Homo sapiens

<400> 26
atggatgcaa tgaagagagg gctctgctgt gtgctgctgc tgtgtggagc agtcttcgtt 60

tcgcccagcc aggaaatcca tgcccgattc agaagaggag ccagatctta ccaaggttgc 120

agcgagccaa ggtgtttcaa cgggggcacc tgccagcagg ccctgtactt ctcagatttc 180
gtgtgccagt gccccgaagg atttgctggg aagtgctgtg aaatagatac cagggccacg 240
tgctacgagg accagggcat cagctacagg ggcacgtgga gcacagcgga gagtggcgcc 300
gagtgcacca actggaacag cagcgcgttg gcccagaagc cctacagcgg gcggaggcca 360
gacgccatca ggctgggcct ggggaaccac aactactgca gaaacccaga tcgagactca 420
aagccctggt gctacgtctt taaggcgggg aagtacagct cagagttctg cagcacccct 480
gcctgctctg agggaaacag tgactgctac tttgggaatg ggtcagccta ccgtggcacg 540
cacagcctca ccgagtcggg tgcctcctgc ctcccgtgga attccatgat cctgataggc 600
aaggtttaca cagcacagaa ccccagtgcc caggcactgg gcctgggcaa acataattac 660
tgccggaatc ctgatgggga tgccaagccc tggtgccacg tgctgaagaa ccgcaggctg 720
acgtgggagt actgtgatgt gccctcctgc tccacctgcg gcctgagaca gtacagccag 780
cctcagtttc gcatcaaagg agggctcttc gccgacatcg cctcccaccc ctggcaggct 840
gccatctttg ccaagcacag gaggtcgccc ggagagcggt tcctgtgcgg gggcatactc 900
atcagctcct gctggattct ctctgccgcc cactgcttcc aggagaggtt tccgccccac 960
cacctgacgg tgatcttggg cagaacatac cgggtggtcc ctggcgagga ggagcagaaa 1020
tttgaagtcg aaaaatacat tgtccataag gaattcgatg atgacactta cgacaatgac 1080
attgcgctgc tgcagctgaa atcggattcg tcccgctgtg cccaggagag cagcgtggtc 1140
cgcactgtgt gccttccccc ggcggacctg cagctgccgg actggacgga gtgtgagctc 1200
tccggctacg gcaagcatga ggccttgtct cctttctatt cggagcggct gaaggaggct 1260
catgtcagac tgtacccatc cagccgctgc acatcacaac atttacttaa cagaacagtc 1320
accgacaaca tgctgtgtgc tggagacact cggagcggcg ggccccaggc aaacttgcac 1380
gacgcctgcc agggcgattc gggaggcccc ctggtgtgtc tgaacgatgg ccgcatgact 1440
ttggtgggca tcatcagctg gggcctgggc tgtggacaga aggatgtccc gggtgtgtac 1500
accaaggtta ccaactacct agactggatt cgtgacaaca tgcgaccgtg a 1551

<210> 27
<211> 1689
<212> DNA
<213> Homo sapiens

<400> 27
atggatgcaa tgaagagagg gctctgctgt gtgctgctgc tgtgtggagc agtcttcgtt 60
tcgcccagcc aggaaatcca tgcccgattc agaagaggag ccagatctta ccaagtgatc 120
tgcagagatg aaaaaacgca gatgatatac cagcaacatc agtcatggct gcgccctgtg 180
ctcagaagca accgggtgga atattgctgg tgcaacagtg gcagggcaca gtgccactca 240
gtgcctgtca aaagttgcag cgagccaagg tgtttcaacg gggcacctg ccagcaggcc 300
ctgtacttct cagatttcgt gtgccagtgc cccgaaggat ttgctgggaa gtgctgtgaa 360

atagatacca gggccacgtg ctacgaggac cagggcatca gctacagggg cacgtggagc 420
acagcggaga gtggcgccga gtgcaccaac tggaacagca gcgcgttggc ccagaagccc 480
tacagcgggc ggaggccaga cgccatcagg ctgggcctgg ggaaccacaa ctactgcaga 540
aacccagatc gagactcaaa gccctggtgc tacgtcttta aggcggggaa gtacagctca 600
gagttctgca gcacccctgc ctgctctgag ggaaacagtg actgctactt tgggaatggg 660
tcagcctacc gtggcacgca cagcctcacc gagtcgggtg cctcctgcct cccgtggaat 720
tccatgatcc tgataggcaa ggtttacaca gcacagaacc ccagtgccca ggcactgggc 780
ctgggcaaac ataattactg ccggaatcct gatggggatg ccaagccctg gtgccacgtg 840
ctgaagaacc gcaggctgac gtgggagtac tgtgatgtgc cctcctgctc cacctgcggc 900
ctgagacagt acagccagcc tcagtttcgc atcaaaggag ggctcttcgc cgacatcgcc 960
tcccacccct ggcaggctgc catctttgcc aagcacagga ggtcgcccgg agagcggttc 1020
ctgtgcgggg gcatactcat cagctcctgc tggattctct ctgccgccca ctgcttccag 1080
gagaggtttc cgccccacca cctgacggtg atcttgggca gaacataccg ggtggtccct 1140
ggcgaggagg agcagaaatt tgaagtcgaa aaatacattg tccataagga attcgatgat 1200
gacacttacg acaatgacat tgcgctgctg cagctgaaat cggattcgtc ccgctgtgcc 1260
caggagagca gcgtggtccg cactgtgtgc cttcccccgg cggacctgca gctgccggac 1320
tggacggagt gtgagctctc cggctacggc aagcatgagg ccttgtctcc tttctattcg 1380
gagcggctga aggaggctca tgtcagactg tacccatcca gccgctgcac atcacaacat 1440
ttacttaaca gaacagtcac cgacaacatg ctgtgtgctg gagacactcg gagcggcggg 1500
ccccaggcaa acttgcacga cgcctgccag ggcgattcgg gaggccccct ggtgtgtctg 1560
aacgatggcc gcatgacttt ggtgggcatc atcagctggg gcctgggctg tggacagaag 1620
gatgtcccgg gtgtgtacac caaggttacc aactacctag actggattcg tgacaacatg 1680
cgaccgtga 1689

<210> 28
<211> 1386
<212> DNA
<213> Homo sapiens

<400> 28
atgtggcagc tcacaagcct cctgctgttc gtggccacct ggggaatttc cggcacacca 60
gctcctcttg actcagtgtt ctccagcagc gagcgtgccc accaggtgct gcggatccgc 120
aaacgtgcca actccttcct ggaggagctc cgtcacagca gcctggagcg ggagtgcata 180
gaggagatct gtgacttcga ggaggccaag gaaatttcc aaaatgtgga tgacacactg 240
gccttctggt ccaagcacgt cgacggtgac cagtgcttgg tcttgccctt ggagcacccg 300
tgcgccagcc tgtgctgcgg gcacggcacg tgcatcgacg gcatcggcag cttcagctgc 360
gactgccgca gcggctggga gggccgcttc tgccagcgcg aggtgagctt cctcaattgc 420
tcgctggaca acggcggctg cacgcattac tgcctagagg aggtgggctg gcggcgctgt 480

agctgtgcgc ctggctacaa gctgggggac gacctcctgc agtgtcaccc cgcagtgaag 540
ttcccttgtg ggaggccctg gaagcggatg gagaagaagc gcagtcacct gaaacgagac 600
acagaagacc aagaagacca agtagatccg cggctcattg atgggaagat gaccaggcgg 660
ggagacagcc cctggcaggt ggtcctgctg gactcaaaga agaagctggc ctgcggggca 720
gtgctcatcc acccctcctg ggtgctgaca gcggcccact gcatggatga gtccaagaag 780
ctccttgtca ggcttggaga gtatgacctg cggcgctggg agaagtggga gctggacctg 840
gacatcaagg aggtcttcgt ccaccccaac tacagcaaga gcaccaccga caatgacatc 900
gcactgctgc acctggccca gcccgccacc ctctcgcaga ccatagtgcc catctgcctc 960
ccggacagcg gccttgcaga gcgcgagctc aatcaggccg gccaggagac cctcgtgacg 1020
ggctggggct accacagcag ccgagagaag gaggccaaga gaaaccgcac cttcgtcctc 1080
aacttcatca agattcccgt ggtcccgcac aatgagtgca gcgaggtcat gagcaacatg 1140
gtgtctgaga acatgctgtg tgcgggcatc ctcggggacc ggcaggatgc ctgcgagggc 1200
gacagtgggg ggcccatggt cgcctccttc cacggcacct ggttcctggt gggcctggtg 1260
agctggggtg agggctgtgg gctccttcac aactacggcg tttacaccaa agtcagccgc 1320
tacctcgact ggatccatgg gcacatcaga gacaaggaag ccccccagaa gagctgggca 1380
ccttag 1386

<210> 29
<211> 1611
<212> DNA
<213> Homo sapiens

<400> 29
atggagtttt caagtccttc cagagaggaa tgtcccaagc ctttgagtag ggtaagcatc 60
atggctggca gcctcacagg attgcttcta cttcaggcag tgtcgtgggc atcaggtgcc 120
cgcccctgca tccctaaaag cttcggctac agctcggtgg tgtgtgtctg caatgccaca 180
tactgtgact cctttgaccc cccgaccttt cctgcccttg gtaccttcag ccgctatgag 240
agtacacgca gtgggcgacg gatggagctg agtatggggc ccatccaggc taatcacacg 300
ggcacaggcc tgctactgac cctgcagcca gaacagaagt tccagaaagt gaagggattt 360
ggaggggcca tgacagatgc tgctgctctc aacatccttg ccctgtcacc ccctgcccaa 420
aatttgctac ttaaatcgta cttctctgaa gaaggaatcg gatataacat catccgggta 480
cccatggcca gctgtgactt ctccatccgc acctacacct atgcagacac ccctgatgat 540
ttccagttgc acaacttcag cctcccagag gaagatacca agctcaagat acccctgatt 600
caccgagccc tgcagttggc ccagcgtccc gtttcactcc ttgccagccc ctggacatca 660
cccacttggc tcaagaccaa tggagcggtg aatgggaagg ggtcactcaa gggacagccc 720
ggagacatct accaccagac ctgggccaga tactttgtga agttcctgga tgcctatgct 780
gagcacaagt tacagttctg ggcagtgaca gctgaaaatg agccttctgc tgggctgttg 840

agtggatacc ccttccagtg cctgggcttc acccctgaac atcagcgaga cttcattgcc 900
cgtgacctag gtcctaccct cgccaacagt actcaccaca atgtccgcct actcatgctg 960
gatgaccaac gcttgctgct gccccactgg gcaaaggtgg tactgacaga cccagaagca 1020
gctaaatatg ttcatggcat tgctgtacat tggtacctgg actttctggc tccagccaaa 1080
gccaccctag gggagacaca ccgcctgttc cccaacacca tgctctttgc ctcagaggcc 1140
tgtgtgggct ccaagttctg ggagcagagt gtgcggctag gctcctggga tcgagggatg 1200
cagtacagcc acagcatcat cacgaacctc ctgtaccatg tggtcggctg gaccgactgg 1260
aaccttgccc tgaaccccga aggaggaccc aattgggtgc gtaactttgt cgacagtccc 1320
atcattgtag acatcaccaa ggacacgttt tacaaacagc ccatgttcta ccaccttggc 1380
cacttcagca agttcattcc tgagggctcc cagagagtgg ggctggttgc cagtcagaag 1440
aacgacctgg acgcagtggc actgatgcat cccgatggct ctgctgttgt ggtcgtgcta 1500
aaccgctcct ctaaggatgt gcctcttacc atcaaggatc ctgctgtggg cttcctggag 1560
acaatctcac ctggctactc cattcacacc tacctgtggc gtcgccagtg a 1611

<210> 30
<211> 1290
<212> DNA
<213> Homo sapiens

<400> 30
atgcagctga ggaacccaga actacatctg ggctgcgcgc ttgcgcttcg cttcctggcc 60
ctcgtttcct gggacatccc tggggctaga gcactggaca atggattggc aaggacgcct 120
accatgggct ggctgcactg ggagcgcttc atgtgcaacc ttgactgcca ggaagagcca 180
gattcctgca tcagtgagaa gctcttcatg gagatggcag agctcatggt ctcagaaggc 240
tggaaggatg caggttatga gtacctctgc attgatgact gttggatggc tccccaaaga 300
gattcagaag gcagacttca ggcagaccct cagcgctttc ctcatgggat tcgccagcta 360
gctaattatg ttcacagcaa aggactgaag ctagggattt atgcagatgt tggaaataaa 420
acctgcgcag gcttccctgg gagttttgga tactacgaca ttgatgccca gacctttgct 480
gactggggag tagatctgct aaaatttgat ggttgttact gtgacagttt ggaaaatttg 540
gcagatggtt ataagcacat gtccttggcc ctgaatagga ctggcagaag cattgtgtac 600
tcctgtgagt ggcctcttta tatgtggccc tttcaaaagc ccaattatac agaaatccga 660
cagtactgca atcactggcg aaattttgct gacattgatg attcctggaa aagtataaag 720
agtatcttgg actggacatc ttttaaccag gagagaattg ttgatgttgc tggaccaggg 780
ggttggaatg acccagatat gttagtgatt ggcaactttg gcctcagctg gaatcagcaa 840
gtaactcaga tggccctctg ggctatcatg gctgctcctt tattcatgtc taatgacctc 900
cgacacatca gccctcaagc caaagctctc cttcaggata aggacgtaat tgccatcaat 960
caggacccct tgggcaagca agggtaccag cttagacagg gagacaactt tgaagtgtgg 1020
gaacgacctc tctcaggctt agcctgggct gtagctatga taaaccggca ggagattggt 1080

ggacctcgct cttataccat cgcagttgct tccctgggta aaggagtggc ctgtaatcct 1140

gcctgcttca tcacacagct cctccctgtg aaaaggaagc tagggttcta tgaatggact 1200

tcaaggttaa gaagtcacat aaatcccaca ggcactgttt tgcttcagct agaaaataca 1260

atgcagatgt cattaaaaga cttactttaa 1290

<210> 31
<211> 2859
<212> DNA
<213> Homo sapiens

<400> 31
atgggagtga ggcacccgcc ctgctcccac cggctcctgg ccgtctgcgc cctcgtgtcc 60

ttggcaaccg ctgcactcct ggggcacatc ctactccatg atttcctgct ggttccccga 120

gagctgagtg gctcctcccc agtcctggag gagactcacc cagctcacca gcagggagcc 180

agcagaccag ggccccggga tgcccaggca caccccggcc gtcccagagc agtgcccaca 240

cagtgcgacg tcccccccaa cagccgcttc gattgcgccc ctgacaaggc catcacccag 300

gaacagtgcg aggcccgcgg ctgttgctac atccctgcaa agcaggggct gcagggagcc 360

cagatggggc agccctggtg cttcttccca cccagctacc ccagctacaa gctggagaac 420

ctgagctcct ctgaaatggg ctacacggcc accctgaccc gtaccacccc caccttcttc 480

cccaaggaca tcctgaccct gcggctggac gtgatgatgg agactgagaa ccgcctccac 540

ttcacgatca aagatccagc taacaggcgc tacgaggtgc ccttggagac cccgcatgtc 600

cacagccggg caccgtcccc actctacagc gtggagttct ccgaggagcc cttcggggtg 660

atcgtgcgcc ggcagctgga cggccgcgtg ctgctgaaca cgacggtggc gcccctgttc 720

tttgcggacc agttccttca gctgtccacc tcgctgccct cgcagtatat cacaggcctc 780

gccgagcacc tcagtcccct gatgctcagc accagctgga ccaggatcac cctgtggaac 840

cgggaccttg cgcccacgcc cggtgcgaac ctctacgggt ctcacccttt ctacctggcg 900

ctggaggacg gcgggtcggc acacggggtg ttcctgctaa acagcaatgc catggatgtg 960

gtcctgcagc cgagccctgc ccttagctgg aggtcgacag gtgggatcct ggatgtctac 1020

atcttcctgg gcccagagcc caagagcgtg gtgcagcagt acctggacgt tgtgggatac 1080

ccgttcatgc cgccatactg gggcctgggc ttccacctgt gccgctgggg ctactcctcc 1140

accgctatca cccgccaggt ggtggagaac atgaccaggg cccacttccc cctggacgtc 1200

cagtggaacg acctggacta catggactcc cggagggact tcacgttcaa caaggatggc 1260

ttccgggact tcccggccat ggtgcaggag ctgcaccagg gcggccggcg ctacatgatg 1320

atcgtggatc ctgccatcag cagctcgggc cctgccggga gctacaggcc ctacgacgag 1380

ggtctgcgga gggggtttt catcaccaac gagaccggcc agccgctgat tgggaaggta 1440

tggcccgggt ccactgcctt ccccgacttc accaacccca cagccctggc ctggtgggag 1500

gacatggtgg ctgagttcca tgaccaggtg cccttcgacg gcatgtggat tgacatgaac 1560

gagccttcca acttcatcag gggctctgag gacggctgcc ccaacaatga gctggagaac 1620
ccaccctacg tgcctggggt ggttgggggg accctccagg cggccaccat ctgtgcctcc 1680
agccaccagt ttctctccac acactacaac ctgcacaacc tctacggcct gaccgaagcc 1740
atcgcctccc acagggcgct ggtgaaggct cgggggacac gcccatttgt gatctcccgc 1800
tcgacctttg ctggccacgg ccgatacgcc ggccactgga cgggggacgt gtggagctcc 1860
tgggagcagc tcgcctcctc cgtgccagaa atcctgcagt ttaacctgct gggggtgcct 1920
ctggtcgggg ccgacgtctg cggcttcctg ggcaacacct cagaggagct gtgtgtgcgc 1980
tggacccagc tgggggcctt ctaccccttc atgcggaacc acaacagcct gctcagtctg 2040
ccccaggagc cgtacagctt cagcgagccg gcccagcagg ccatgaggaa ggccctcacc 2100
ctgcgctacg cactcctccc ccacctctac acactgttcc accaggccca cgtcgcgggg 2160
gagaccgtgg cccggcccct cttcctggag ttccccaagg actctagcac ctggactgtg 2220
gaccaccagc tcctgtgggg ggaggccctg ctcatcaccc cagtgctcca ggccgggaag 2280
gccgaagtga ctggctactt ccccttgggc acatggtacg acctgcagac ggtgccagta 2340
gaggcccttg gcagcctccc acccccacct gcagctcccc gtgagccagc catccacagc 2400
gaggggcagt gggtgacgct gccggccccc ctggacacca tcaacgtcca cctccgggct 2460
gggtacatca tccccctgca gggccctggc ctcacaacca cagagtcccg ccagcagccc 2520
atggccctgg ctgtggccct gaccaagggt ggggaggccc gaggggagct gttctgggac 2580
gatggagaga gcctggaagt gctggagcga ggggcctaca cacaggtcat cttcctggcc 2640
aggaataaca cgatcgtgaa tgagctggta cgtgtgacca gtgagggagc tggcctgcag 2700
ctgcagaagg tgactgtcct gggcgtggcc acggcgcccc agcaggtcct ctccaacggt 2760
gtccctgtct ccaacttcac ctacagcccc gacaccaagg tcctggacat ctgtgtctcg 2820
ctgttgatgg gagagcagtt tctcgtcagc tggtgttag 2859

<210> 32
<211> 1296
<212> DNA
<213> Homo sapiens

<400> 32
atgcacgtgc gctcactgcg agctgcggcg ccgcacagct tcgtggcgct ctgggcaccc 60
ctgttcctgc tgcgctccgc cctggccgac ttcagcctgg acaacgaggt gcactcgagc 120
ttcatccacc ggcgcctccg cagccaggag cggcgggaga tgcagcgcga gatcctctcc 180
attttgggct tgccccaccg cccgcgcccg cacctccagg gcaagcacaa ctcggcaccc 240
atgttcatgc tggacctgta caacgccatg gcggtggagg agggcggcgg gcccggcggc 300
cagggcttct cctaccccta caaggccgtc ttcagtaccc agggcccccc tctggccagc 360
ctgcaagata gccatttcct caccgacgcc gacatggtca tgagcttcgt caacctcgtg 420
gaacatgaca aggaattctt ccacccacgc taccaccatc gagagttccg gtttgatctt 480
tccaagatcc cagaagggga agctgtcacg gcagccgaat tccggatcta caaggactac 540

atccgggaac gcttcgacaa tgagacgttc cggatcagcg tttatcaggt gctccaggag 600
cacttgggca gggaatcgga tctcttcctg ctcgacagcc gtaccctctg ggcctcggag 660
gagggctggc tggtgtttga catcacagcc accagcaacc actgggtggt caatccgcgg 720
cacaacctgg gcctgcagct ctcggtggag acgctggatg ggcagagcat caaccccaag 780
ttggcgggcc tgattgggcg gcacgggccc cagaacaagc agcccttcat ggtggctttc 840
ttcaaggcca cggaggtcca cttccgcagc atccggtcca cggggagcaa acagcgcagc 900
cagaaccgct ccaagacgcc caagaaccag gaagccctgc ggatggccaa cgtggcagag 960
aacagcagca gcgaccagag gcaggcctgt aagaagcacg agctgtatgt cagcttccga 1020
gacctgggct ggcaggactg gatcatcgcg cctgaaggct acgccgccta ctactgtgag 1080
ggggagtgtg ccttccctct gaactcctac atgaacgcca ccaaccacgc catcgtgcag 1140
acgctggtcc acttcatcaa cccggaaacg gtgcccaagc cctgctgtgc gcccacgcag 1200
ctcaatgcca tctccgtcct ctacttcgat gacagctcca acgtcatcct gaagaaatac 1260
agaaacatgg tggtccgggc ctgtggctgc cactag 1296

<210> 33
<211> 1191
<212> DNA
<213> Homo sapiens

<400> 33
atggtggccg ggacccgctg tcttctagcg ttgctgcttc cccaggtcct cctgggcggc 60
gcggctggcc tcgttccgga gctgggccgc aggaagttcg cggcggcgtc gtcgggccgc 120
ccctcatccc agccctctga cgaggtcctg agcgagttcg agttgcggct gctcagcatg 180
ttcggcctga aacagagacc cacccccagc agggacgccg tggtgccccc ctacatgcta 240
gacctgtatc gcaggcactc aggtcagccg ggctcacccg ccccagacca ccggttggag 300
agggcagcca gccgagccaa cactgtgcgc agcttccacc atgaagaatc tttggaagaa 360
ctaccagaaa cgagtgggaa aacaacccgg agattcttct ttaatttaag ttctatcccc 420
acggaggagt ttatcacctc agcagagctt caggttttcc gagaacagat gcaagatgct 480
ttaggaaaca atagcagttt ccatcaccga attaatattt atgaaatcat aaaacctgca 540
acagccaact cgaaattccc cgtgaccaga cttttggaca ccaggttggt gaatcagaat 600
gcaagcaggt gggaaagttt tgatgtcacc cccgctgtga tgcggtggac tgcacaggga 660
cacgccaacc atggattcgt ggtggaagtg gcccacttgg aggagaaaca aggtgtctcc 720
aagagacatg ttaggataag caggtctttg caccaagatg aacacagctg gtcacagata 780
aggccattgc tagtaacttt tggccatgat ggaaaagggc atcctctcca caaaagagaa 840
aaacgtcaag ccaaacacaa acagcggaaa cgccttaagt ccagctgtaa gagacaccct 900
ttgtacgtgg acttcagtga cgtggggtgg aatgactgga ttgtggctcc cccggggtat 960
cacgcctttt actgccacgg agaatgccct tttcctctgg ctgatcatct gaactccact 1020

aatcatgcca ttgttcagac gttggtcaac tctgttaact ctaagattcc taaggcatgc 1080

tgtgtcccga cagaactcag tgctatctcg atgctgtacc ttgacgagaa tgaaaaggtt 1140

gtattaaaga actatcagga catggttgtg gagggttgtg ggtgtcgcta g 1191

<210> 34
<211> 1962
<212> DNA
<213> Homo sapiens

<400> 34
atgcgtcccc tgcgcccccg cgccgcgctg ctggcgctcc tggcctcgct cctggccgcg 60

cccccggtgg ccccggccga ggccccgcac ctggtgcatg tggacgcggc ccgcgcgctg 120

tggcccctgc ggcgcttctg gaggagcaca ggcttctgcc ccccgctgcc acacagccag 180

gctgaccagt acgtcctcag ctgggaccag cagctcaacc tcgcctatgt gggcgccgtc 240

cctcaccgcg gcatcaagca ggtccggacc cactggctgc tggagcttgt caccaccagg 300

gggtccactg gacggggcct gagctacaac ttcacccacc tggacgggta cctggacctt 360

ctcagggaga accagctcct cccagggttt gagctgatgg gcagcgcctc gggccacttc 420

actgactttg aggacaagca gcaggtgttt gagtggaagg acttggtctc cagcctggcc 480

aggagataca tcggtaggta cggactggcg catgtttcca agtggaactt cgagacgtgg 540

aatgagccag accaccacga ctttgacaac gtctccatga ccatgcaagg cttcctgaac 600

tactacgatg cctgctcgga gggtctgcgc gccgccagcc ccgccctgcg gctgggaggc 660

cccggcgact ccttccacac cccaccgcga tccccgctga gctggggcct cctgcgccac 720

tgccacgacg gtaccaactt cttcactggg gaggcgggcg tgcggctgga ctacatctcc 780

ctccacagga agggtgcgcg cagctccatc tccatcctgg agcaggagaa ggtcgtcgcg 840

cagcagatcc ggcagctctt ccccaagttc gcggacaccc ccatttacaa cgacgaggcg 900

gacccgctgg tgggctggtc cctgccacag ccgtggaggg cggacgtgac ctacgcggcc 960

atggtggtga aggtcatcgc gcagcatcag aacctgctac tggccaacac cacctccgcc 1020

ttcccctacg cgctcctgag caacgacaat gccttcctga gctaccaccc gcaccccttc 1080

gcgcagcgca cgctcaccgc gcgcttccag gtcaacaaca cccgcccgcc gcacgtgcag 1140

ctgttgcgca agccggtgct cacggccatg gggctgctgg cgctgctgga tgaggagcag 1200

ctctgggccg aagtgtcgca ggccgggacc gtcctggaca gcaaccacac ggtgggcgtc 1260

ctggccagcg cccaccgccc ccagggcccg gccgacgcct ggcgcgccgc ggtgctgatc 1320

tacgcgagcg acgacacccg cgcccacccc aaccgcagcg tcgcggtgac cctgcggctg 1380

cgcggggtgc cccccggccc gggcctggtc tacgtcacgc gctacctgga caacgggctc 1440

tgcagccccg acggcgagtg gcggcgcctg ggccggcccg tcttccccac ggcagagcag 1500

ttccggcgca tgcgcgcggc tgaggacccg gtggccgcgg cgcccccgccc cttacccgcc 1560

ggcggccgcc tgaccctgcg ccccgcgctg cggctgccgt cgcttttgct ggtgcacgtg 1620

tgtgcgcgcc ccgagaagcc gcccgggcag gtcacgcggc tccgcgccct gcccctgacc 1680

caagggcagc tggttctggt ctggtcggat gaacacgtgg gctccaagtg cctgtggaca 1740

tacgagatcc agttctctca ggacggtaag gcgtacaccc cggtcagcag gaagccatcg 1800

accttcaacc tctttgtgtt cagcccagac acaggtgctg tctctggctc ctaccgagtt 1860

cgagccctgg actactgggc ccgaccaggc cccttctcgg accctgtgcc gtacctggag 1920

gtccctgtgc caagagggcc cccatccccg ggcaatccat ga 1962

<210> 35
<211> 1398
<212> DNA
<213> Homo sapiens

<400> 35
atgctgccac tttggactct ttcactgctg ctgggagcag tagcaggaaa agaagtttgc 60

tacgaaagac tcggctgctt cagtgatgac tccccatggt caggaattac ggaaagaccc 120

ctccatatat tgccttggtc tccaaaagat gtcaacaccc gcttcctcct atatactaat 180

gagaacccaa acaactttca agaagttgcc gcagattcat caagcatcag tggctccaat 240

ttcaaaacaa atagaaaaac tcgctttatt attcatggat tcatagacaa gggagaagaa 300

aactggctgg ccaatgtgtg caagaatctg ttcaaggtgg aaagtgtgaa ctgtatctgt 360

gtggactgga aaggtggctc ccgaactgga tacacacaag cctcgcagaa catcaggatc 420

gtgggagcag aagtggcata ttttgttgaa tttcttcagt cggcgttcgg ttactcacct 480

tccaatgtgc atgtcattgg ccacagcctg ggtgcccacg ctgctgggga ggctggaagg 540

agaaccaatg ggaccattgg acgcatcaca gggttggacc cagcagaacc ttgctttcag 600

ggcacacctg aattagtccg attggacccc agcgatgcca aatttgtgga tgtaattcac 660

acggatggtg cccccatagt ccccaatttg gggtttggaa tgagccaagt cgtgggccac 720

ctagatttct ttccaaatgg aggagtggaa atgcctggat gtaaaaagaa cattctctct 780

cagattgtgg acatagacgg aatctgggaa gggactcgag actttgcggc ctgtaatcac 840

ttaagaagct acaaatatta cactgatagc atcgtcaacc ctgatggctt tgctggattc 900

ccctgtgcct cttacaacgt cttcactgca aacaagtgtt tcccttgtcc aagtggaggc 960

tgcccacaga tgggtcacta tgctgataga tatcctggga aacaaatgga tgtgggccag 1020

aaattttatc tagacactgg tgatgccagt aattttgcac gttggaggta taaggtatct 1080

gtcacactgt ctggaaaaaa ggttacagga cacatactag tttctttgtt cggaaataaa 1140

ggaaactcta agcagtatga aattttcaag ggcactctca aaccagatag tactcattcc 1200

aatgaatttg actcagatgt ggatgttggg gacttgcaga tggttaaatt tatttggtat 1260

aacaatgtga tcaacccaac tttacctaga gtgggagcat ccaagattat agtggagaca 1320

aatgttggaa aacagttcaa cttctgtagt ccagaaaccg tcagggagga agttctgctc 1380

accctcacac cgtgttag 1398

<210> 36

<211> 1536
<212> DNA
<213> Homo sapiens

<400> 36
atgaagttct ttctgttgct tttcaccatt gggttctgct gggctcagta ttccccaaat 60
acacaacaag gacggacatc tattgttcat ctgtttgaat ggcgatgggt tgatattgct 120
cttgaatgtg agcgatattt agctccgaag ggatttggag gggttcaggt ctctccacca 180
aatgaaaatg ttgcaattta caaccctttc agaccttggt gggaaagata ccaaccagtt 240
agctataaat tatgcacaag atctggaaat gaagatgaat ttagaaacat ggtgactaga 300
tgtaacaatg ttggggttcg tatttatgtg gatgctgtaa ttaatcatat gtgtggtaac 360
gctgtgagtg caggaacaag cagtacctgt ggaagttact tcaaccctgg aagtagggac 420
tttccagcag tcccatattc tggatgggat ttcaatgatg gtaaatgtaa aactggaagt 480
ggagatatcg agaactacaa tgatgctact caggtcagag attgtcgtct gactggtctt 540
cttgatcttg cactggagaa ggattacgtg cgttctaaga ttgccgaata tatgaaccat 600
ctcattgaca ttggtgttgc agggttcaga cttgatgctt ccaagcacat gtggcctgga 660
gacataaagg caattttgga caaactgcat aatctaaaca gtaactggtt ccctgcagga 720
agtaaacctt tcatttacca ggaggtaatt gatctgggtg gtgagccaat taaaagcagt 780
gactactttg gtaatggccg ggtgacagaa ttcaagtatg gtgcaaaact cggcacagtt 840
attcgcaagt ggaatggaga gaagatgtct tacttaaaga actggggaga aggttggggt 900
ttcgtacctt ctgacagagc gcttgtcttt gtggataacc atgacaatca acgaggacat 960
ggggctggag gagcctctat tcttaccttc tgggatgcta ggctgtacaa aatggcagtt 1020
ggatttatgc ttgctcatcc ttacggattt acacgagtaa tgtcaagcta ccgttggcca 1080
agacagtttc aaaatggaaa cgatgttaat gattgggttg ggccaccaaa taataatgga 1140
gtaattaaag aagttactat taatccagac actacttgtg gcaatgactg ggtctgtgaa 1200
catcgatggc gccaaataag gaacatggtt attttccgca atgtagtgga tggccagcct 1260
tttacaaatt ggtatgataa tgggagcaac caagtggctt ttgggagagg aaacagagga 1320
ttcattgttt tcaacaatga tgactggtca ttttctttaa ctttgcaaac tggtcttcct 1380
gctggcacat actgtgatgt catttctgga gataaaatta atggcaattg cacaggcatt 1440
aaaatttacg tttctgatga tggcaaagct cattttttcta ttagtaactc tgctgaagat 1500
ccatttattg caattcatgc tgaatctaaa ttgtaa 1536

<210> 37
<211> 1536
<212> DNA
<213> Homo sapiens

<400> 37
atgaagttct ttctgttgct tttcaccatt gggttctgct gggctcagta ttccccaaat 60
acacaacaag gacggacatc tattgttcat ctgtttgaat ggcgatgggt tgatattgct 120

cttgaatgtg agcgatattt agctcccaag ggatttggag gggttcaggt ctctccacca 180
aatgaaaatg ttgcaattca caacccttc agaccttggt gggaaagata ccaaccagtt 240
agctataaat tatgcacaag atctggaaat gaagatgaat ttagaaacat ggtgactaga 300
tgtaacaatg ttggggttcg tatttatgtg gatgctgtaa ttaatcatat gtctggtaat 360
gctgtgagtg caggaacaag cagtacctgt ggaagttact tcaaccctgg aagtagggac 420
tttccagcag tcccatattc tggatgggat tttaatgatg gtaaatgtaa aactggaagt 480
ggagatatcg agaactacaa tgatgctact caggtcagag attgtcgtct ggttggtctt 540
cttgatcttg cactggagaa agattatgtg cgttccaaga ttgccgaata tatgaatcat 600
ctcattgaca ttggtgttgc agggttcaga cttgatgctt ccaagcacat gtggcctgga 660
gacataaagg caattttgga caaactgcat aatctaaaca gtaactggtt ccctgcagga 720
agtaaacctt tcatttacca ggaggtaatt gatctgggtg gtgagccaat taaaagcagt 780
gactactttg gaaatggccg ggtgacagaa ttcaagtatg gtgcaaaact cggcacagtt 840
attcgcaagt ggaatggaga gaagatgtct tacctaaaga actggggaga aggttggggt 900
ttcatgcctt ctgacagagc acttgtcttt gtggataacc atgacaatca acgaggacat 960
ggggctggag gagcctctat tcttaccttc tgggatgcta ggctgtataa aatggcagtt 1020
ggatttatgc ttgctcatcc ttatggtttt acacgagtaa tgtcaagcta ccgttggcca 1080
agacagtttc aaaatggaaa cgatgttaat gattgggttg ggccaccaaa taataatgga 1140
gtaattaaag aagttactat taatccagac actacttgtg gcaatgactg ggtctgtgaa 1200
catcgatggc gccaaataag gaacatggtt aatttccgca atgtagtgga tggccagcct 1260
tttacaaact ggtatgataa tgggagcaac caagtggctt ttgggagagg aaacagagga 1320
ttcattgttt tcaacaatga tgactggaca ttttctttaa ctttgcaaac tggtcttcct 1380
gctggcacat actgtgatgt catttctgga gataaaatta atggcaattg cacaggcatt 1440
aaaatctacg tttctgacga tggcaaagct catttttcta ttagtaactc tgctgaggat 1500
ccatttattg caattcatgc tgaatctaaa ttataa 1536

<210> 38
<211> 1197
<212> DNA
<213> Homo sapiens

<400> 38
atgtggctgc ttttaacaat ggcaagtttg atatctgtac tggggactac acatggtttg 60
tttggaaaat tacatcctgg aagccctgaa gtgactatga acattagtca gatgattact 120
tattggggat acccaaatga agaatatgaa gttgtgactg aagatggtta tattcttgaa 180
gtcaatagaa ttccttatgg gaagaaaaat tcagggaata caggccagag acctgttgtg 240
tttttgcagc atggtttgct tgcatcagcc acaaactgga tttccaacct gccgaacaac 300
agccttgcct tcattctggc agatgctggt tatgatgtgt ggctgggcaa cagcagagga 360
aacacctggg ccagaagaaa cttgtactat tcaccagatt cagttgaatt ctgggctttc 420

agctttgatg aaatggctaa atatgacctt ccagccacaa tcgacttcat tgtaaagaaa 480

actggacaga agcagctaca ctatgttggc cattcccagg gcaccaccat tggttttatt 540

gccttttcca ccaatcccag cctggctaaa agaatcaaaa ccttctatgc tctagctcct 600

gttgccactg tgaagtatac aaaaagcctt ataaacaaac ttagatttgt tcctcaatcc 660

ctcttcaagt ttatatttgg tgacaaaata ttctacccac acaacttctt tgatcaattt 720

cttgctactg aagtgtgctc ccgtgagatg ctgaatctcc tttgcagcaa tgccttattt 780

ataatttgtg gatttgacag taagaacttt aacacgagtc gcttggatgt gtatctatca 840

cataatccag caggaacttc tgttcaaaac atgttccatt ggacccaggc tgttaagtct 900

gggaaattcc aagcttatga ctggggaagc ccagttcaga ataggatgca ctatgatcag 960

tcccaacctc cctactacaa tgtgacagcc atgaatgtac caattgcagt gtggaacggt 1020

ggcaaggacc tgttggctga cccccaagat gttggccttt tgcttccaaa actccccaat 1080

cttatttacc acaaggagat tcctttttac aatcacttgg actttatctg ggcaatggat 1140

gcccctcaag aagtttacaa tgacattgtt tctatgatat cagaagataa aaagtag 1197

<210> 39
<211> 1830
<212> DNA
<213> Homo sapiens

<400> 39
atgaagtggg taacctttat ttcccttctt tttctcttta gctcggctta ttccaggggt 60

gtgtttcgtc gagatgcaca caagagtgag gttgctcatc ggtttaaaga tttgggagaa 120

gaaaatttca aagccttggt gttgattgcc tttgctcagt atcttcagca gtgtccattt 180

gaagatcatg taaaattagt gaatgaagta actgaatttg caaaaacatg tgttgctgat 240

gagtcagctg aaaattgtga caaatcactt catacccttt ttggagacaa attatgcaca 300

gttgcaactc ttcgtgaaac ctatggtgaa atggctgact gctgtgcaaa acaagaacct 360

gagagaaatg aatgcttctt gcaacacaaa gatgacaacc caaacctccc ccgattggtg 420

agaccagagg ttgatgtgat gtgcactgct tttcatgaca atgaagagac atttttgaaa 480

aaatacttat atgaaattgc cagaagacat ccttactttt atgccccgga actccttttc 540

tttgctaaaa ggtataaagc tgcttttaca gaatgttgcc aagctgctga taaagctgcc 600

tgcctgttgc caaagctcga tgaacttcgg gatgaaggga aggcttcgtc tgccaaacag 660

agactcaagt gtgccagtct ccaaaaattt ggagaaagag ctttcaaagc atgggcagta 720

gctcgcctga gccagagatt tcccaaagct gagtttgcag aagtttccaa gttagtgaca 780

gatcttacca aagtccacac ggaatgctgc catggagatc tgcttgaatg tgctgatgac 840

agggcggacc ttgccaagta tatctgtgaa aatcaagatt cgatctccag taaactgaag 900

gaatgctgtg aaaaacctct gttggaaaaa tcccactgca ttgccgaagt ggaaaatgat 960

gagatgcctg ctgacttgcc ttcattagct gctgattttg ttgaaagtaa ggatgtttgc 1020

aaaaactatg ctgaggcaaa ggatgtcttc ctgggcatgt ttttgtatga atatgcaaga 1080
aggcatcctg attactctgt cgtgctgctg ctgagacttg ccaagacata tgaaaccact 1140
ctagagaagt gctgtgccgc tgcagatcct catgaatgct atgccaaagt gttcgatgaa 1200
tttaaacctc ttgtggaaga gcctcagaat ttaatcaaac aaaattgtga gctttttgag 1260
cagcttggag agtacaaatt ccagaatgcg ctattagttc gttacaccaa gaaagtaccc 1320
caagtgtcaa ctccaactct tgtagaggtc tcaagaaacc taggaaaagt gggcagcaaa 1380
tgttgtaaac atcctgaagc aaaaagaatg ccctgtgcag aagactatct atccgtggtc 1440
ctgaaccagt tatgtgtgtt gcatgagaaa acgccagtaa gtgacagagt caccaaatgc 1500
tgcacagaat ccttggtgaa caggcgacca tgcttttcag ctctggaagt cgatgaaaca 1560
tacgttccca aagagtttaa tgctgaaaca ttcaccttcc atgcagatat atgcacactt 1620
tctgagaagg agagacaaat caagaaacaa actgcacttg ttgagctcgt gaaacacaag 1680
cccaaggcaa caaaagagca actgaaagct gttatggatg atttcgcagc ttttgtagag 1740
aagtgctgca aggctgacga taaggagacc tgctttgccg aggagggtaa aaaacttgtt 1800
gctgcaagtc aagctgcctt aggcttataa 1830

<210> 40
<211> 990
<212> DNA
<213> Homo sapiens

<400> 40
gcaagcttca agggcccatc ggtcttcccc ctggcaccct cctccaagag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacatctgca acgtgaatca caagcccagc aacaccaagg tggacaagaa agttgagccc 300
aaatcttgtg acaaaactca cacatgccca ccgtgcccag cacctgaact cctgggggga 360
ccgtcagtct tcctcttccc cccaaaaccc aaggacaccc tcatgatctc ccggacccct 420
gaggtcacat gcgtggtggt ggacgtgagc cacgaagacc ctgaggtcaa gttcaactgg 480
tacgtggacg gcgtggaggt gcataatgcc aagacaaagc cgcgggagga gcagtacaac 540
agcacgtacc gtgtggtcag cgtcctcacc gtcctgcacc aggactggct gaatggcaag 600
gagtacaagt gcaaggtctc caacaaagcc ctcccagccc ccatcgagaa aaccatctcc 660
aaagccaaag ggcagccccg agaaccacag gtgtacaccc tgcccccatc ccgggatgag 720
ctgaccaaga accaggtcag cctgacctgc ctggtcaaag gcttctatcc cagcgacatc 780
gccgtggagt gggagagcaa tgggcagccg gagaacaact acaagaccac gcctcccgtg 840
ctggactccg acggctcctt cttcctctac agcaagctca ccgtggacaa gagcaggtgg 900
cagcagggga acgtcttctc atgctccgtg atgcatgagg ctctgcacaa ccactacacg 960
cagaagagcc tctccctgtc tccgggtaaa 990

<210> 41
<211> 981
<212> DNA
<213> Homo sapiens

<400> 41
gcaagcttca agggcccatc ggtcttcccc ctggtgccct gctccaggag cacctccgag 60

agcacagccg ccctgggctg cctggtcaag gactacttcc ccgaaccggt gacggtgtcg 120

tggaactcat gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttgagtcc 300

aaatatggtc ccccatgccc atcatgccca gcacctgagt tcctgggggg accatcagtc 360

ttcctgttcc ccccaaaacc caaggacact ctcatgatct cccggacccc tgaggtcacg 420

tgcgtggtgg tggacgtgag ccaggaagac cccgaggtcc agttcaactg gtacgtggat 480

ggcgtggagg tgcataatgc caagacaaag ccgcgggagg agcagttcaa cagcacgtac 540

cgtgtggtca gggtcctcac cgtcctgcac caggactggc tgaacggtaa ggagtacaag 600

tgcaaggtct ccaacaaagg cctcccgtcc tccatcgaga aaaccatctc caaagccaaa 660

gggcagcccc gagagccaca ggtgtacacc ctgcccccat cccaggagga gatgaccaag 720

aaccaggtca gcctgacctg cctggtcaaa ggcttctacc ccagcgacat cgccgtggag 780

tgggagagca atgggcagcc ggaggacaac tacaagacca cgcctcccgt gctggactcc 840

gacggctcct tcttcctcta cagcaggcta accgtggaca agagcaggtg gcaggagggg 900

aatgtcttct catgctccgt gatgcatgag gctctgcaca accactacac acagaagagc 960

ctctccctgt ctccgggtaa a 981

<210> 42
<211> 349
<212> DNA
<213> Homo sapiens

<400> 42
atggagtttg ggctgagctg gctttttctt gtggctattt taaaaggtgt ccagtgtgag 60

gtgcagctgt tggagtctgg gggaggcttg gtacagcctg gggggtccct gagactctcc 120

tgtgcagcct ctggattcac ctttagcagc tatgccatga gctgggtccg ccagtctcca 180

gggaaggggc tacagtgggt ctcagctatt agtggtagtg gtattagcac atactacgca 240

gactccgtga ggggccggtt caccatctcc agagacaatt ccaagaacac gctgtatctg 300

caaatgagca gcctgagccg aggacacggc cgtatattac tgtgcgaaa 349

<210> 43
<211> 711
<212> DNA
<213> Homo sapiens

<400> 43

atggacatga gggtccccgc tcagctcctg gggctcctgc tgctctggct cccaggtgcc 60
agatgtgtca tctggatgac ccagtctcca tccttactct ctgcatctac gggagacaga 120
gtcacaatca gttgtcggat gagtcagggc attagcaatt atttagcctg gtatcagcaa 180
aaaccaggga aagcccctga cctcctgatc tatgctgcat ccactttgca aagtggggtc 240
ccatcaaggt tcagtggcag tggatctggg acagatttca ttctcaccat cagccgcctg 300
cagtctgaag attttgcaat ttattactgt caacagtatt atagtttccc attcactttc 360
ggccctggga ccaaagtgga tatcaaacga actgtggctg caccatctgt cttcatcttc 420
ccgccatctg atgagcagtt gaaatctgga actgcctctg ttgtgtgcct gctgaataac 480
ttctatccca gagaggccaa agtacagtgg aaggtggata acgccctcca atcgggtaac 540
tcccaggaga gtgtcacaga gcaggacagc aaggacagca cctacagcct cagcagcacc 600
ctgacgctga gcaaagcaga ctacgagaaa cacaaagtct acgcctgcga agtcacccat 660
cagggcctga gctcgcccgt cacaaagagc ttcaacaggg gagagtgtta g 711

<210> 44
<211> 558
<212> DNA
<213> Gaussia princeps

<400> 44
atgggagtga aagttctttt tgcccttatt tgtattgctg tggccgaggc caaaccaact 60
gaaaacaatg aagatttcaa cattgtagct gtagctagca actttgctac aacggatctc 120
gatgctgacc gtggtaaatt gcccggaaaa aaattaccac ttgaggtact caaagaaatg 180
gaagccaatg ctaggaaagc tggctgcact aggggatgtc tgatatgcct gtcacacatc 240
aagtgtacac ccaaaatgaa gaagtttatc ccaggaagat gccacaccta tgaaggagac 300
aaagaaagtg cacagggagg aataggagag gctattgttg acattcctga aattcctggg 360
tttaaggatt tggaacccat ggaacaattc attgcacaag ttgacctatg tgtagactgc 420
acaactggat gcctcaaagg tcttgccaat gtgcaatgtt ctgatttact caagaaatgg 480
ctgccacaaa gatgtgcaac ttttgctagc aaaattcaag gccaagtgga caaaataaag 540
ggtgccggtg gtgattaa 558

<210> 45
<211> 507
<212> DNA
<213> Gaussia princeps

<400> 45
atgccaactg aaaacaatga agatttcaac attgtagctg tagctagcaa ctttgctaca 60
acggatctcg atgctgaccg tggtaaattg cccggaaaaa aattaccact tgaggtactc 120
aaagaaatgg aagccaatgc taggaaagct ggctgcacta ggggatgtct gatatgcctg 180
tcacacatca agtgtacacc caaaatgaag aagtttatcc caggaagatg ccacacctat 240
gaaggagaca aagaaagtgc acagggagga ataggagagg ctattgttga cattcctgaa 300

attcctgggt ttaaggattt ggaacccatg gaacaattca ttgcacaagt tgacctatgt 360

gtagactgca caactggatg cctcaaaggt cttgccaatg tgcaatgttc tgatttactc 420

aagaaatggc tgccacaaag atgtgcaact tttgctagca aaattcaagg ccaagtggac 480

aaaataaagg gtgccggtgg tgattaa 507

<210> 46
<211> 19
<212> DNA
<213> Artificial sequence

<220>
<223> Primer

<400> 46
cattgtagct gtagctagc 19

<210> 47
<211> 17
<212> DNA
<213> Artificial sequence

<220>
<223> Primer

<400> 47
ttaatcacca ccggcac 17

<210> 48
<211> 579
<212> DNA
<213> Mus musculus

<400> 48
atgggggtgc ccgaacgtcc caccctgctg cttttactct ccttgctact gattcctctg 60

ggcctcccag tcctctgtgc tcccccacgc ctcatctgcg acagtcgagt tctggagagg 120

tacatcttag aggccaagga ggcagaaaat gtcacgatgg gttgtgcaga aggtcccaga 180

ctgagtgaaa atattacagt cccagatacc aaagtcaact tctatgcttg gaaaagaatg 240

gaggtggaag aacaggccat agaagtttgg caaggcctgt ccctgctctc agaagccatc 300

ctgcaggccc aggccctgct agccaattcc tcccagccac cagagaccct tcagcttcat 360

atagacaaag ccatcagtgg tctacgtagc ctcacttcac tgcttcgggt actgggagct 420

cagaaggaat tgatgtcgcc tccagatacc accccacctg ctccactccg aacactcaca 480

gtggatactt tctgcaagct cttccgggtc tacgccaact tcctccgggg gaaactgaag 540

ctgtacacgg gagaggtctg caggagaggg gacaggtga 579

<210> 49
<211> 504
<212> DNA
<213> Mus musculus

<400> 49
atggctcccc cacgcctcat ctgcgacagt cgagttctgg agaggtacat cttagaggcc 60

aaggaggcag aaaatgtcac gatgggttgt gcagaaggtc ccagactgag tgaaaatatt 120

acagtcccag ataccaaagt caacttctat gcttggaaaa gaatggaggt ggaagaacag 180

gccatagaag tttggcaagg cctgtccctg ctctcagaag ccatcctgca ggcccaggcc 240

ctgctagcca attcctccca gccaccagag acccttcagc ttcatataga caaagccatc 300

agtggtctac gtagcctcac ttcactgctt cgggtactgg gagctcagaa ggaattgatg 360

tcgcctccag ataccacccc acctgctcca ctccgaacac tcacagtgga tactttctgc 420

aagctcttcc gggtctacgc caacttcctc cgggggaaac tgaagctgta cacgggagag 480

gtctgcagga gaggggacag gtga 504

<210> 50
<211> 21
<212> DNA
<213> Artificial sequence

<220>
<223> Primer

<400> 50
cacgatgggt tgtgcagaag g 21

<210> 51
<211> 22
<212> DNA
<213> Artificial sequence

<220>
<223> Primer

<400> 51
cgaagcagtg aagtgaggct ac 22

<210> 52
<211> 405
<212> DNA
<213> Homo sapiens

<400> 52
atggcaccta cttcaagttc tacaaagaaa acacagctac aactggagca tttactgctg 60

gatttacaga tgattttgaa tggaattaat aattacaaga atcccaaact caccaggatg 120

ctcacattta agttttacat gcccaagaag gccacagaac tgaaacatct tcagtgtcta 180

gaagaagaac tcaaacctct ggaggaagtg ctaaatttag ctcaaagcaa aaactttcac 240

ttaagaccca gggacttaat cagcaatatc aacgtaatag ttctggaact aaagggatct 300

gaaacaacat tcatgtgtga atatgctgat gagacagcaa ccattgtaga atttctgaac 360

agatggatta ccttttgtca aagcatcatc tcaacactga cttga 405

<210> 53
<211> 768
<212> DNA
<213> Artificial sequence

<220>

<223> chimeric enhanced GFP

<400> 53
atgggagtga aagttctttt tgcccttatt tgtattgctg tggccgaggc cgtgagcaag 60
ggcgaggagc tgttcaccgg ggtggtgccc atcctggtcg agctggacgg cgacgtaaac 120
ggccacaagt tcagcgtgtc cggcgagggc gagggcgatg ccacctacgg caagctgacc 180
ctgaagttca tctgcaccac cggcaagctg cccgtgccct ggcccaccct cgtgaccacc 240
ctgacctacg gcgtgcagtg cttcagccgc taccccgacc acatgaagca gcacgacttc 300
ttcaagtccg ccatgcccga aggctacgtc caggagcgca ccatcttctt caaggacgac 360
ggcaactaca agacccgcgc cgaggtgaag ttcgagggcg acaccctggt gaaccgcatc 420
gagctgaagg gcatcgactt caaggaggac ggcaacatcc tggggcacaa gctggagtac 480
aactacaaca gccacaacgt ctatatcatg gccgacaagc agaagaacgg catcaaggtg 540
aacttcaaga tccgccacaa catcgaggac ggcagcgtgc agctcgccga ccactaccag 600
cagaacaccc ccatcggcga cggccccgtg ctgctgcccg acaaccacta cctgagcacc 660
cagtccgccc tgagcaaaga ccccaacgag aagcgcgatc acatggtcct gctggagttc 720
gtgaccgccg ccgggatcac tctcggcatg gacgagctgt acaagtaa 768

<210> 54
<211> 786
<212> DNA
<213> Artificial sequence

<220>
<223> chimeric enhanced GFP with an Histidin Tag

<400> 54
atgggagtga aagttctttt tgcccttatt tgtattgctg tggccgaggc cgtgagcaag 60
ggcgaggagc tgttcaccgg ggtggtgccc atcctggtcg agctggacgg cgacgtaaac 120
ggccacaagt tcagcgtgtc cggcgagggc gagggcgatg ccacctacgg caagctgacc 180
ctgaagttca tctgcaccac cggcaagctg cccgtgccct ggcccaccct cgtgaccacc 240
ctgacctacg gcgtgcagtg cttcagccgc taccccgacc acatgaagca gcacgacttc 300
ttcaagtccg ccatgcccga aggctacgtc caggagcgca ccatcttctt caaggacgac 360
ggcaactaca agacccgcgc cgaggtgaag ttcgagggcg acaccctggt gaaccgcatc 420
gagctgaagg gcatcgactt caaggaggac ggcaacatcc tggggcacaa gctggagtac 480
aactacaaca gccacaacgt ctatatcatg gccgacaagc agaagaacgg catcaaggtg 540
aacttcaaga tccgccacaa catcgaggac ggcagcgtgc agctcgccga ccactaccag 600
cagaacaccc ccatcggcga cggccccgtg ctgctgcccg acaaccacta cctgagcacc 660
cagtccgccc tgagcaaaga ccccaacgag aagcgcgatc acatggtcct gctggagttc 720
gtgaccgccg ccgggatcac tctcggcatg gacgagctgt acaagcacca ccatcaccac 780
cattaa 786

## Description

### FIELD OF THE INVENTION

The present invention is directed to methods for producing recombinant proteins in diatoms, said polypeptides being secreted in the liquid culture medium.

### BACKGROUND OF THE INVENTION

The present invention relates to the production of recombinant proteins in diatoms. There is a high demand for these recombinant proteins in various domains such as biopharmaceuticals used in therapeutic applications or enzymes used as biocatalysts for industrial processes. As described by the international patent application WO 2009/101160, microalgae are an expression system of choice for the production of recombinant glycosylated proteins over alternative systems such as bacteria, yeast, fungi, plants or animals. Indeed, microalgae are able to perform complex glycosylation of interest. Microalgae present also the advantage of being cultivated in confined photobioreactors or conventionnal fermentors, therefore overcoming the problem of gene dissemination into the environment. In addition, microalgae cultures provide excellent yield in biomass in a short time and only requires synthetic sea water or fresh water, a total chemically defined media, as well as ligh or a carbon source for heterotrophic growing algae.

When producing recombinant proteins, one has to address the purification of them which is often tedious. However, this process can be greatly facilitated by the secretion of the protein in the culture broth. By reducing the number of steps to achieve suitable purity of the products, this leads to an improvement of the overall cost-effectiveness.

In eukaryotes, secreted proteins are translocated across the endoplasmic reticulum (ER) membrane, through the Golgi apparatus and subsequently released in the extracellular medium by secretory vesicles. The protein to be secreted is first produced with an amino-terminal located signal peptide which targets the polypeptide to the endosecretory pathway. This signal peptide is necessary to address the polypeptide to the endoplasmic reticulum and sufficient to lead to the secretion of the aforementioned protein to the extracellular media. During the translocation in the ER/Golgi, the signal peptide is cleaved and the protein is being matured (undergo post translational modifications). It allows the delivery in the culture media of complex mature proteins.

Traditionally, signal peptides are viewed as being functional across species based on their shared characteristics in eukaryotes. For example, human or plant signal sequences can successfully lead to the secretion of recombinant proteins when used in the yeast *Pichia pastoris.* In plant, studies revealed that murine signal peptide sequences can also be functional. Nevertheless, data in the litterature proved that this assumption could not be further from the truth. For example, 4 proteins (VSG 117, VSG MVAT7, VSG 221 and BiP) from *Trypanosoma brucei* and one protein (gp63) from *Leishmania sp.* harboring signal peptide were not translocated into dog pancreatic microsomes used to mimic the passage into the ER membrane (Al-Qahtani *et al*., 1998). Similarly, signal peptide of the carboxypeptidase Y from the yeast *Saccharomyces cerevisiae* did not led to the translocation into the ER of this recombinant protein when expressed in the mammalian COS-1 cells (Bird *et al*., 1987).

To date, no study has been realized to test whether an exogenous signal peptide could lead to the secretion of recombinant proteins in microalgae, and especially in diatoms. Indeed, inferring the secretion machinery based on prior knowledge is hampered by the phylogenetic distance of these microalgae which belong to a eukaryotic phylum faraway from other organisms such as animals. As a member of the eukaryotic lineage Chromalveolates, diatoms are evolutionarily distinct from the plantae, the lineage containing land plants, green and red algae and the opisthokonta containing fungi and metazoa as shown in figure 1 (Keeling *et al*., 2005). A broad gene analysis has revealed major differences in the diatom *P. tricornutum,* when compared to plantae and opisthokonta. Thus, amongst the 3710 gene families identified in *P. tricornutum,* nearly 40% could not be found in plantae and/or opisthokonta (Bowler *et al*., 2008).

### SUMMARY OF THE INVENTION

In a first aspect, the present invention provides a transformed diatom comprising a nucleic acid sequence operatively linked to a promoter, wherein said nucleic acid sequence encodes an amino acid sequence comprising :

(i) an heterologous signal peptide; and
(ii) a polypeptide,
said heterologous signal peptide leading to the secretion of said polypeptide in the extracellular medium of said transformed diatom.

In a preferred embodiment, the transformed diatom is selected from the group comprising Bacillariophyceae diatoms.

In a most preferred embodiment, the transformed diatom is *Phaeodactylum tricornutum.*

In a second aspect, the present invention relates to a method for producing a polypeptide which is secreted in the extracellular medium, said method comprising the steps of:

(i) culturing a transformed diatom as defined previously;
(ii) harvesting the extracellular medium of said culture; and
(iii) purifying the secreted polypeptide in said extracellular medium.

In a third aspect, the present invention refers to the use of a transformed diatom for the secretion of a polypeptide in the extracellular medium.

**BRIEF DESCRIPTION OF DRAWINGS**

Figure 1. Diatoms Phylogeny

Figure 2. Normalized secreted Luciferase activity of *Phaeodactylum tricornutum* transformants.

Figure 3. Detection of secreted Gaussia Luciferase byWestern Blot.

Figure 4. Detection of secreted Erythropoietin by Western Blot.

**DETAILED DESCRIPTION OF THE INVENTION**

The invention aims to provide a new system for producing recombinant polypeptides in a diatom, said polypeptides being secreted in the liquid culture medium.

The applicant surprisingly found that transformed diatoms were capable of producing and secreting a polypeptide in their extracellular media, when being transformed with a sequence encoding a polypeptide and a heterologous signal peptide.

An object of the invention is a transformed diatom comprising a nucleic acid sequence operatively linked to a promoter, wherein said nucleic acid sequence encodes an amino acid sequence comprising:

(i) an heterologous signal peptide; and
(ii) a polypeptide,
said heterologous signal peptide leading to the secretion of said polypeptide in the extracellular medium of said transformed diatom.

The term "nucleic acid sequence" used herein refers to DNA sequences (e.g., cDNA or genomic or synthetic DNA) and RNA sequences (e. g., mRNA or synthetic RNA), as well as analogs of DNA or RNA containing non-natural nucleotide analogs, non-native internucleoside bonds, or both. Preferably, said nucleic acid sequence is a DNA sequence. The nucleic acid can be in any topological conformation, like linear or circular.

"Operatively linked" promoter refers to a linkage in which the promoter is contiguous with the gene of interest to control the expression of said gene.

Examples of promoter that drives expression of a polypeptide in transformed diatoms include, but are not restricted to, nuclear promoters such as fcpA and fcpB from *Phaeodactylum tricornutum* (Zavlaskaïa et al. (2000) Transformation of the diatom Phaeodactylum tricornutum (Bacillariophyceae) with a variety of selectable marker and reporter genes. J Phycol. 36, 379-386).

Transformation of diatoms can be carried out by conventional methods such as microparticles bombardment, electroporation, glass beads, polyethylene glycol (PEG). Such a protocol is disclosed in the examples.

In an embodiment of the invention, nucleotide sequences may be introduced into diatoms *via* a plasmid, virus sequences, double or simple strand DNA, circular or linear DNA.

In another embodiment of the invention, it is generally desirable to include into each nucleotide sequences or vectors at least one selectable marker to allow selection of diatoms that have been stably transformed. Examples of such markers are antibiotic resistant genes such as sh ble gene enabling resistance to zeocin, nat or sat-1 genes enabling resistance to nourseothricin.

After transformation of diatoms, transformants producing the desired proteins secreted in the culture media are selected. Selection can be carried out by one or more conventional methods comprising: enzyme-linked immunosorbent *assay* (ELISA), mass spectroscopy such as MALDI-TOF-MS, ESI-MS chromatography, spectrophotometer, fluorimeter, immunocytochemistry by exposing cells to an antibody having a specific affinity for the desired protein.

The term "polypeptide" as used herein refers to an amino acid sequence comprising amino acids which are linked by peptide bonds. A polypeptide may be monomeric or polymeric. Furthermore, a polypeptide may comprise a number of different domains each of which has one or more distinct activities.

The term "peptide" as used herein refers to an amino acid sequence that is typically less than 50 amino acids long and more typically less than 30 amino acids long.

The term "signal peptide" as used herein refers to an amino acid sequence which is generally located at the amino terminal end of the amino acid sequence of a polypeptide. The signal peptide mediates the translocation of said polypeptide through the secretion pathway and leads to the secretion of said polypeptide in the extracellular medium.

As used herein, the term "secretion pathway" refers to the process used by a cell to secrete proteins out of the intracellular compartment. Such pathway comprises a step of translocation of a polypeptide across the endoplasmic reticulum membrane, followed by the transport of the polypeptide in the Golgi apparatus, said polypeptide being subsequently released in the extracellular medium of the cell by secretory vesicles. Post-translational modifications necessary to obtain mature proteins, such as glycosylation or disulfide bonds formation, are operated on proteins during said secretion pathway.

Preferably, the signal peptide leading to the secretion of the polypeptide in the extracellular medium is located at its amino-terminal end.

This signal peptide is typically 15-30 amino acids long, and presents a 3 domains structure (von Heijne G. (1990) The signal Peptide, J Membr Biol, 115:195-201; Emanuelsson O. et al (2007) Locating proteins in the cell using TargetP, SignalP and related tools. Nat Protoc 2:953-971), which are as follows:

(i) an N-terminal region (n-region) containing positively charged amino acids, such as Arginine (R), Histidine (H) or Lysine (K);
(ii) a central hydrophobic region (h-region) of at least 6 amino acids containing hydrophobic amino acids such as Alanine (A), Cysteine (C), Glycine (G), Isoleucine (I), Leucine (L), Methionine (M), Phenylalanine (F), Proline (P), Tryptophan (W) or Valine (V); and
(iii) a C-terminal region (c-region) of polar uncharged amino acids such as Asparagine (R), Glutamine (Q), Serine (S), Threonine (T) or Tyrosine (Y). Said C-region often contains a helix-breaking proline or glycine that helps define a cleavage site. Small uncharged residues in positions -3 and -1 (defined as the number of residue before the cleavage site) are usually requires for an efficient cleavage by signal peptidase following the translocation across the endoplasmic reticulum membrane (von Heijne G. (1990) The signal Peptide, J Membr Biol 115:195-201; Vernet K., Schatz G. (1988) Protein translocation across membranes, Science, 241:1307-1313).

A person skilled in the art is able to simply identify a signal peptide in an amino acid sequence, for example by using the SignalP 3.0 Server (accessible on line at http://www.cbs.dtu.dk/services/SignalP/) which predicts the presence and location of signal peptide cleavage sites in amino acid sequences from different organisms by using two different models: the Neural networks and the Hidden Markov models (Emanuelsson O. et al (2007) Locating proteins in the cell using TargetP, SignalP and related tools. Nat Protoc 2:953-971).

The term "heterologous", with reference to a signal peptide or to a polypeptide, means an amino acid sequence which does not exist in the corresponding diatom before its transformation. It is intended that the term encompasses proteins that are encoded by wild-type genes, mutated genes, and/or synthetic genes.

In a preferred embodiment, the polypeptide secreted in the extracellular medium of transformed diatoms according to the invention is a heterologous polypeptide.

Advantageously, the heterologous signal peptide used herein corresponds to the signal peptide of said heterologous polypeptide, said signal peptide leading to the secretion of said heterologous polypeptide in the extracellular medium of the cell from which it is originate. An example of such embodiment is disclosed in the examples, wherein the signal peptide leading to the secretion of *Gaussia princeps* luciferase in *P. tricornutum* is its native signal peptide.

In a still preferred embodiment, said heterologous polypeptide which is secreted in the extracellular medium of the transformed diatom according to the invention can be of animal origin. Preferably, said polypeptide is of mammalian origin. Most preferably, said polypeptide is of human origin. Examples of such embodiment in the present invention include the murine erythropoietin and the human interleukin-2.

In another preferred embodiment, the polypeptide to be secreted in the extracellular medium of the transformed diatoms of the invention is a protein of therapeutic interest selected in the group comprising antibodies and their fragments, erythropoietin, cytokines such as interferons, coagulation factors, hormones, beta-glucocerebrosidase, pentraxin-3, anti-TNFs, $\alpha$-glucosidase acide, $\alpha$-L-iduronidase and derivatives thereof.

An antibody is an immunoglobulin molecule corresponding to a tetramer comprising four polypeptide chains, two identical heavy (H) chains (about 50-70 kDa when full length) and two identical light (L) chains (about 25 kDa when full length) inter-connected by disulfide bonds. Light chains are classified as kappa and lambda. Heavy chains are classified as gamma, mu, alpha, delta, or epsilon, and define the antibody's isotype as IgG, IgM, IgA, IgD, and IgE, respectively. Each heavy chain is comprised of an amino-terminal heavy chain variable region (abbreviated herein as HCVR) and a heavy chain constant region. The heavy chain constant region is comprised of three domains (CH1, CH2, and CH3) for IgG, IgD, and IgA; and 4 domains (CH1, CH2, CH3, andCH4) for IgM and IgE. Each light chain is comprised of an amino-terminal light chain variable region (abbreviated herein as LCVR) and a light chain constant region. The light chain constant region is comprised of one domain, CL. The HCVR and LCVR regions can be further subdivided into regions of hypervariability, termed complementarity determining regions (CDRs), interspersed with regions that are more conserved, termed framework regions (FR). Each HCVR and LCVR is composed of three CDRs and four FRs, arranged from amino-terminus to carboxy-terminus in the following order: FR1, CDR1, FR2, CDR2, FR3, CDR3, FR4. The assignment of amino acids to each domain is in accordance with well-known conventions. The functional ability of the antibody to bind a particular antigen depends on the variable regions of each light/heavy chain pair, and is largely determined by the CDRs.

The term "antibody", as used herein, refers to a monoclonal antibody *per se.* A monoclonal antibody can be a human antibody, chimeric antibody and/or humanized antibody.

The term "antibody fragments" as used herein refers to antibody fragments that bind to the particular antigens of said antibody. For example, antibody fragments capable of binding to particular antigens include Fab (e.g., by papain digestion), Fab' (e.g., by pepsin digestion and partial reduction) and F(ab')2 (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the invention.

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(ab')2 heavy chain portion can be designed to include DNA sequences encoding the CH1 domain and/or hinge region of the heavy chain. The various portions of antibodies can be joined together chemically by conventional techniques, or can be prepared as a contiguous protein using genetic engineering techniques.

The term "Cytokines" refers to signaling proteins which are released by specific cells of the immune system to carry a signal to other cells in order to alter their function. Cytokines are immunomodulating agents and are extensively used in cellular communication. The term cytokines encompasses a wide range of polypeptide regulators, such as interferons, interleukins, chemokins or Tumor Necrosis Factor.

The term "Coagulation factors" refers to the plasma proteins which interact with platelets in a complex cascade of enzyme-catalyzed reactions, leading to the formation of fibrin for the initiation of a blood clot in the blood coagulation process. Coagulation factors, at the number of 13, are generally serine proteases, but also comprise glycoproteins (Factors VIII and V) or others types of enzyme, such as transglutaminase (Factor XIII).

The term "Hormones" refers to chemical messengers secreted by specific cells in the plasma or the lymph to produce their effects on other cells of the organism at a distance from their production sites. Most hormones initiate a cellular response by initially combining with either a specific intracellular or cell membrane associated receptor protein. Common known hormones are, for example, insulin for the regulation of energy and glucose in the organism, or the Growth Hormone which stimulates growth and cell reproduction and regeneration.

As used herein, the term "derivative" refers to a polypeptide having a percentage of identity of at least 90% with the complete amino acid sequence of any of the protein of therapeutic interest disclosed previously and having the same activity.

Preferably, a derivative has a percentage of identity of at least 95% with said amino acid sequence, and preferably of at least 99% with said amino acid sequence.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized by using computer softwares using algorithms such as GAP, BESTFIT, BLAST P, BLAST N, FASTA, TFASTA in the Wisconsin Genetics software Package, Genetics Computer Group, 575 Science Dr., Madison, WI USA. To get the best local alignment, one can preferably used BLAST software, with the BLOSUM 62 matrix, preferably the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

In a most preferred embodiment of the invention, the nucleic acid sequence encoding an amino acid sequence comprising (i) an heterologous signal peptide and (ii) a polypeptide, said heterologous signal peptide leading to the secretion of said polypeptide in the extracellular medium of diatoms, are selected in the group comprising the sequences disclosed in Table I.

**Table I**

| PROTEIN | Accession numbers (CDS) | CDS SEQ ID N° | Accession number (Protein) | Comments |
|---|---|---|---|---|
| **Interferons** | | | | |
| Interferon β 1 | CCDS6495 | SEQ ID N° 1 | NP_002167 | |
| Interferon β2 | CCDS6506 | SEQ ID N° 2 | NP_000596 | |
| **Interleukins** | | | | |
| IL-11 | CCDS 12923 | SEQ ID № 3 | NP_000632 | |
| IL-6= Interferon β 2 | CCDS5375 | SEQ ID N° 5 | NP_000591 | |
| IL-21 | CCDS3727 | SEQ ID N° 6 | NP_068575 | |
| **Hormones** | | | | |
| Insulin | J00265 | SEQ ID№ 7 | AAA59172 | |
| Preproglucagon Variants | V01515 | SEQ ID N° 8 | CAA24759 | |
| EPO | CCDS5705 | SEQ ID N° 9 | NP_000790 | |
| Growth hormone | CCDS 11653 | SEQ ID N° 10 | NP_000506 | isoform 1 |
| | CCDS45760 | SEQ № 11 | NP_072053 | isoform 2 |
| | CCDS11654 | SEQ ID N° 12 | NP_072054 | isoform 3 |
| | CCDS42371 | SEQ ID N° 13 | NP_072055 | isoform 4 |
| | NM 022562 | SEQ ID N° 14 | NP_072056 | isoform 5 |
| GM-CSF(colony stimulating factor 2 granulocyte-macrophage) | CCDS4150 | SEQ ID N° 15 | NP_000749 | |
| G-CSF (Granulocyte-Colony stimulating Factor 3 | CCDS11357 | SEQ ID N° 16 | NP_000750 | isoform a |
| Follicle stimulating hormone | CCDS5007 | SEQ ID N° 17 | NP_000726 | subunit alpha |
| | CCDS7868 | SEQ ID N° 18 | NP_000501 | subunit beta |
| Chorionic gonadotropin | CCDS5007 | SEQ ID N° 17 | NP_000726 | subunit alpha |
| | CCDS12749 | SEQ ID N° 19 | NP_000728 | subunit beta |
| Thyroid stimulating hormone (Thyrogen) | CCDS5007 | SEQ ID N° 17 | NP_000726 | subunit alpha |
| | CCDS880 | SEQ ID N° 20 | NP_000540 | subunit beta |
| Luteinizing hormone | CCDS5007 | SEQ ID N° 17 | NP_000726 | subunit alpha |
| | CCDS 12748 | SEQ ID N° 21 | NP_000885 | subunit beta |
| **Coagulation factors** | | | | |
| Factor II =thrombin | CCDS31476 | SEQ ID N° 22 | NP_000497 | |

(continued)

| PROTEIN | Accession numbers (CDS) | CDS SEQ ID N° | Accession number (Protein) | Comments |
|---|---|---|---|---|
| Factor VII | J02933 | SEQ ID N° 23 | AAA51983 | |
| Factor VIII | K01740 | SEQ ID N° 24 | AAA52484 | |
| Factor IX | J00136 | SEQ ID N° 25 | AAA98726 | |
| Tissue plasminogen | CCDS6127 | SEQ ID N° 26 | NP_127509 | isoform 3 |
| activator | CCDS6126 | SEQ ID N° 27 | NP_000921 | isoform 1 |
| Protein C | CCDS2145 | SEQ ID N° 28 | NP_000303 | |
| **Lysosomal enzymes** | | | | |
| β-glucocerebrosidase = β-glucosidase acid | CCDS1102 | SEQ ID N° 29 | NP_000148 | |
| α-Galactosidase A | CCDS 14484 | SEQ ID N° 30 | NP_000160 | |
| Alglucosidase = α-glucosidase acid | CCDS32760 | SEQ ID N° 31 | NP_000143 | |
| **Other proteins** | | | | |
| Bone morphogenetic protein 7 =osteogenic protein-1 | CCDS 13455 | SEQ ID N° 32 | NP_001710 | |
| Bone morphogenetic protein 2 | CCDS 13099 | SEQ ID N° 33 | NP_001191 | |
| α-L-iduronidase | CCDS3343 | SEQ ID N° 34 | NP_000194 | |
| Pancreatic lipase | CCDS7594 | SEQ ID N° 35 | NP_000927 | |
| Pancreatic amylases | CCDS783 | SEQ ID N° 36 | NP_000690 | α-2A-amylase |
| | CCDS782 | SEQ ID N° 37 | NP_066188 | α-2B-amylase |
| Gastric lipase | CCDS7389 | SEQ ID N° 38 | NP_004181 | |
| Albumin | CCDS3555 | SEQ ID N° 39 | NP_000468 | |
| **Antibodies** | | | | |
| immunoglobulin heavy chain constant region gamma | AJ294730 | SEQ ID N° 40 | CAC20454 | Gamma 1 |
| | AJ294733 | SEQ ID N° 41 | CAC20457 | Gamma 4 |
| Immunoglobulin Variable Heavy Chain | M26995 | SEQ ID N° 42 | AAA59127 | |
| Immunoglobulin Kappa light Chain (VL+CL) | AJ010442 | SEQ ID N° 43 | CAA09181 | |

In another preferred embodiment, the polypeptide to be secreted in the extracellular medium of the transformed diatom of the invention is a protein allowing modifications of said diatom to improve its industrial application. Examples of such embodiment include the secretion by microalgae of enzymes in the extracellular media to modify its own cell wall in order to improve biodegradability and therefore biomass conversion efficiency for applications such as biofuels. Enzymes to be produced for hydrolysis of microalgal cell wall oligosaccharides into soluble sugars include, but are not limited to, mannosidases or galactosidases. In another example of such embodiment, enzymes secreted in the media allow the modification of cell wall to enhance adsorption ability of microalgae on solid support. Applications of such technology include immobilization of microalgae for used as biocatalyst, biosensor or in bioremediation processes.

In another embodiment of this invention, polypeptides to be produced in the extracellular media are ligninolytic enzymes used in green chemistry. Examples of these enzymes include, but are not limited to, lignin peroxidases, manganese-dependant peroxidases and laccases. By improving the biodegradability of wood material, these enzymes have biotechnological applications in biopulping and biofuel production from plant origin. These enzymes can also be used to treat industrial waste such as polluted water containing toxic dyes from the textile industry.

Another embodiment of this invention is the genetic engineering of optimal biomaterials based on microalgal carbohydrate polymers. An example of enzymes to be secreted in the media for such applications includes peroxidases such as horseradish peroxidase allowing the cross-linking of tyramine-conjugated polymers to form hydrogel. In another example of this application, the enzyme to be secreted in the media is a transglutaminases to perform cross-linking of proteins of interest onto the sugar backbone of carbohydrate polymers.

The term "enzyme", when used herein refers to a molecule having at least one enzymatic activity, and includes full-length enzymes, catalytically active fragments, chimerics, complexes, and the like. A "catalytically active fragment" of an enzyme refers to a polypeptide having a detectable level of functional (enzymatic) activity.

Host cells used herein for the secretion of a polypeptide in the extracellular medium are aquatic photosynthetic microorganism which belongs to Bacillariophyceae also known as Diatoms.

In a most preferred embodiment, the diatom is *Phaeodactylum tricornutum..*

In another embodiment of the invention, diatoms used herein for the secretion of polypeptides in the extracellular medium further express an N-acetylglucosaminyltransferase (GnT I, GnT II, GnT III, GnT IV, GnT V or GnT VI), a mannosidase II and a fucosyltransferase, galactosyltransferase (GalT) or sialyltransferases (ST), to secrete glycosylated polypeptides. Glycosylation is dependent on the endogenous machinery present in the host cell chosen for producing and secreting glycosylated polypeptides. Diatoms are capable of producing such glycosylated polypeptides in high yield via their endogenous N-glycosylation machinery.

Another object of the invention is a method for producing a polypeptide which is secreted in the extracellular medium, said method comprising the steps of:

(i) culturing a transformed diatom as described above;
(ii) harvesting the extracellular medium of said culture; and
(iii) purifying the polypeptide, which is secreted in said extracellular medium.

In another embodiment of the invention, the method of producing a polypeptide which is secreted in the extracellular medium of diatoms comprises a former step of transforming said diatoms with a nucleic acid sequence operatively linked to a promoter, wherein said nucleic acid sequence encodes an amino acid sequence comprising an heterologous signal peptide and a polypeptide, said heterologous signal peptide leading to the secretion of said polypeptide in the extracellular medium of said transformed diatom.

In another embodiment of the invention, the method of producing secreted polypeptide in the extracellular medium of transformed diatoms further comprises a step (iv) of determining the glycosylation pattern of said polypeptide.

Preliminary information about N-glycosylation of the recombinant polypeptide secreted in the extracellular medium can be obtained by affino- and immunoblotting analysis using specific probes such as lectins (CON A; ECA; SNA; MAA...) and specific N-glycans antibodies (anti-1,2-xylose; anti--1,3-fucose; anti-Neu5Gc, anti-Lewis ...). To investigate the detailed N-glycan profile of recombinant polypeptide, N-linked oligosaccharides is released from the polypeptide in a non specific manner using enzymatic digestion or chemical treatment. The resulting mixture of reducing oligosaccharides can be profiled by HPLC and/or mass spectrometry approaches (ESI-MS-MS and MALDI-TOF essentially). These strategies, coupled to exoglycosidase digestion, enable N-glycan identification and quantification (Séveno et al., 2008, Plant N-glycan profiling of minute amounts of material, Anal. Biochem., vol.379 (1), p :66-72; Stadlmann et al., 2008, Analysis of immunoglobulin glycosylation by LC-ESI-MS of glycopeptides and oligosaccharides. Proteomics, vol. 8, p :2858-2871).

In a preferred embodiment, the method of producing a polypeptide secreted in the extracellular medium of diatoms leads to the secretion of at least 25%, 50%, 75% or 90% of the polypeptide expressed in said diatoms.

Secretion efficiency can be assessed using pulse-chase experiments with radiolabeled amino acids, as described by Jensen *et al.* (2000), except that media are replaced by those used to grow diatoms. The protein to study is then immunoprecipitated on both intracellular and extracellular fractions and subjected to SDS-PAGE electrophoresis and quantified using the phosphor-imaging technology.

The percentage of secretion for any given time can be calculated as follow:

$$\text{QSecreted} + \text{Qinternal} = 100\% \text{ of expressed polypeptides}$$

$$\% \text{ secreted} = (\text{Qsecreted} \times 100\%) / (\text{Qsecreted} + \text{Qinternal})$$

Said formula can be merely explained as following :

- quantity of the polypeptide of interest in the extracellular medium of transformed diatoms (Qsecreted);
- quantity of said polypeptide in the intracellular medium of transformed diatoms (Qinternal)
- Additionning both quantities as determined precedently to obtain the total quantity of produced polypeptides by the transformed diatoms, such quantity being equivalent to 100% (100% of expressed polypeptides)
- Multiplying the amount of secreted polypeptides (Qsecreted) by 100%, and dividing the result by the total of polypeptides expressed by the transformed diatoms (Qsecreted+Qinternal) to obtain the percentage of polypeptides secreted in the extracellular medium of said diatoms (%secreted).

Another object of the invention is the use of a transformed diatom as previously described for the secretion of a polypeptide in the extracellular medium.

In the following, the invention is described in more detail with reference to methods. Yet, no limitation of the invention is intended by the details of the examples. Rather, the invention pertains to any embodiment which comprises details which are not explicitly mentioned in the examples herein, but which the skilled person finds without undue effort.

## EXAMPLES

### Example 1 Secretion of *Gaussia princeps* luciferase in the culture medium of transformed *Phaeodactylum tricornutum*

To test the functionality of an exogenous signal peptide, *Phaeodactylum tricornutum (P. Tricornutum)* was transformed with a plasmid containing *Gaussia princeps* luciferase (GLuc) coding sequence. This luciferase is responsible for the bioluminescent reaction of the marine copepod *Gaussia princeps.* Its amino terminal extremity carried a signal peptide leading to the natural secretion of the enzyme in the extracellular medium. The whole native GLuc sequence including the signal peptide from *G. princeps* was used to transform P. *tricornutum.* As a control, *P. tricornutum* was also transformed with the GLuc sequence lacking the signal peptide as determined using SignalP.

a) <u>Standard culture conditions of Phaeodactylum tricornutum</u>

Strains used in this work were *Phaeodactylum tricornutum.* Diatoms were grown at 20°C under continuous illumination (280-350 $\mu$mol photons.m$^{-2}$.s$^{-1}$), in natural coastal seawater sterilized by 0.22 $\mu$m filtration. This seawater is enriched with nutritive Conway media (Walne, 1966) with addition of silica (40 mg/L of sodium metasilicate). For large volume (from 2 liters to 300 liters) cultures were aerated with a 2% $CO_2$/air mixture to maintain the pH in a range of 7.5-8.1.

For genetic transformation, diatoms were spread on gelose containing 1% of agar. After concentration by centrifugation, the diatoms were spread on petri dishes sealed and incubated at 20°C under constant illumination. Concentration of cultures was estimated on Mallassez counting cells after fixation of the microalgae with a Lugol's solution.

b) <u>Expression constructs for GLuc</u>

The cloning vector pPHA-T1 built by Zavlaskaïa *et al.* (2000) includes sequences of *P. tricornutum* promoters fcpA and fcpB (fucoxanthin-chlorophyll a/c-binding proteins A and B) and the terminator of fcpA. It contains a selection cassette with the gene she ble and a MCS flanking the fcpA promoter. *Gaussia* luciferase is encoded by a 558 pb sequence (SEQ ID N°44). The full lentgth *Gaussia* luciferase coding sequence was synthesized with the addition of EcoRI and HindIII restriction sites flanking the 5' and 3' ends respectively. As a control, a *Gaussia* luciferase coding sequence lacking the signal peptide was also synthetized (SEQ ID N°45) with EcoRI and HindIII restriction sites at both ends. After digestion by EcoRI and HindIII, both inserts were introduced into pPHA-T1 vectors. A vector lacking the luciferase coding sequence was used as control.

c) <u>Genetic transformation</u>

The transformation was carried out by particles bombardment using the BIORAD PDS-1000/He apparatus modified by Thomas JL. et al. (2001) A helium burst biolistic device adapted to penetrate fragile insect tissues, Journal of Insect Science, 1-9*).*

Cultures of diatoms *(P. tricornutum*) in exponential growth phase were concentrated by centrifugation (10 minutes, 2150 g, 20°C), diluted in sterile seawater, and spread on geloses at $10^8$ cells per dish. The microcarriers were gold particles (diameter 0.6 $\mu$m). Microcarriers were prepared according to the protocol of the supplier (BIORAD). Parameters used for shooting were the following:

- use of the long nozzle,
- use of the stopping ring with the largest hole,
- 15 cm between the stopping ring and the target (diatoms cells),
- precipitation of the DNA with 1.25 M $CaCl_2$ and 20 mM spermidine,
- a ratio of 1.25 μg DNA for 0.75mg gold particles per shot,
- rupture disk of 900 psi with a distance of escape of 0.2 cm,
- a vacuum of 30H g

Diatoms were incubated 24 hours before the addition of the antibiotic zeocin (100μg/ml) and were then maintained at 20°C under constant illumination. After 1-2 weeks of incubation of the plates, individual clones were picked from the plates and inoculated into liquid medium containing zeocin (100μg/ml).

d) Microalgae DNA extraction

Cells ($5.10^8$) transformed by the vector bearing the full-length GLuc, GLuc lacking the signal peptide or control plasmid were pelleted by centrifugation (2150g, 15 minutes, 4°C). Microalgae cells were incubated overnight at 4°C with 4mL of TE NaCl 1X buffer (Tris-HCL 0.1 M, EDTA 0.05 M, NaCl 0.1 M, pH 8). 1% SDS, 1% Sarkosyl and 0.4 $mg.mL^{-1}$ of proteinase K were then added to the sample, followed by an incubation at 40°C for 90 minutes. A first phenol-chloroform isoamyl alcohol extraction was carried out to extract an aqueous phase comprising the nucleic acids. RNA presents in the sample was eliminated by an hour incubation at 60°C in the presence of RNase (1 $\mu g.mL^{-1}$). A second phenol-chloroform extraction was carried out, followed by a precipitation a precipitation with ethanol. Finally, the pellet was dried and solubilised into 200 μL of ultrapure sterile water. Quantification of DNA was carried out by spectrophotometry (260 nm) and analysed by agarose gel electrophoresis.

e) Polymerase chain reaction (PCR) analysis

The incorporation of the heterologous full-length GLuc and Gluc lacking the signal peptide in the genome of *Phaeodactylum tricornutum* was assessed by PCR analysis. The sequence of primers used for the amplification of GLuc transformed cells were 5'-CATTGTAGCTGTAGCTAGC-3' (SEQ ID N° 46) and 5'-TTAATCACCACCGGCAC-3'(SEQ ID N° 47). The PCR reaction was carried out in a final volume of 50 μl consisting of 1X PCR buffer, 0.2 mM of each dNTP, 5 μM of each primer, 20 ng of template DNA and 1.25 U of *Taq* DNA polymerase (Taq DNA polymerase, ROCHE). Thirty cycles were conducted for amplification of template DNA. Initial denaturation was performed at 94°C for 4 min. Each subsequent cycle consisted of a 94°C (1 min) melting step, a 55°C (1 min) annealing step, and a 72°C (1 min) extension. Samples obtained after the PCR reaction were run on agarose gel (1%) stained with ethidium bromide.

Results revealed a single band at 478bp for cells transformed with the constructs carrying the full-length GLuc or Gluc lacking its signal peptide (data not shown). No band was detected in cells transformed with the control vector. This result validates the incorporation of exogenous gene in the genome of *Phaeodactylum tricornutum.*

f) Luciferase activity

GLuc catalyzes the oxidative decarboxylation of coelenterazine to produce the excited state of coelenteramide, which upon relaxation to the ground state emits light. This enzymatic property was used to test the presence and functionality of GLuc in *P. tricornutum.*

The luciferase activity was measured in the culture medium of transformants harboring the full-length GLuc (92 cell lines), GLuc lacking its signal peptide (90 cell lines) as well as cells transformed with the control vector (96 cell lines). A 96 wells microplate luminometer with automated substrate injection was used (Victor™ X3, Perkin Elmer). The coelenterazine substrate (Luxinnovate) was resuspended in acidic ethanol at a concentration of 5 mg/mL and this stock solution was stored at -80°C. Prior to measurements, a working solution of substrate was prepared by diluting the stock solution in distilled water (1:300). This solution was kept at room temperature for 20 minutes before the start of the experiment. *P. tricornutum* transformed with the full-length *Gaussia* luciferase or lacking the signal peptide as well as wild-type cells were grown in 96 wells microplate and centrifuged (10 minutes, 2150g, 20°C) at exponential phase of growth. Forty μL of culture supernatant was then mixed with 40 μL of the coelenterazine working solution using automated injection and shaking. Light emission was recorded for 10 seconds.

Cells transformed with the full-length GLuc sequence were classified into 5 groups depending on their luciferase activity (figure 2). Variable levels of luciferase activity were detected in the full-length GLuc transformants tested ranging from signals corresponding to the background (i.e. <1000 light units) to signals above $1.10^6$ light units. This wide distribution is typically observed for non-homologous transformation of the nuclear genome. Indeed, the number of transgene copies inserted in the nuclear genome and/or the location in the genome can vary between clones resulting in variable level of transgene expression. No luciferase activity above the background was detected for cells transformed with GLuc lacking the signal peptide or control cells (data not shown). Altogether, these results confirm the functionality in *P. tricornutum* of the native signal peptide of GLuc from *G. princeps.* Furthermore, it also demonstrates the functionality of the luciferase in term of enzymatic activity.

g) Immunoblotting analysis

Wild-type and transformed cells were cultured and the corresponding culture medium were separated from cells and subsequently concentrated by flow filtration.

Aliquotes of wild-type and transformed cells of *P. tricornutum* culture at exponential phase of growth were collected and cells were separated from the culture medium by centrifugation (10 minutes, 2150g, 20°C). The supernatant was filtered using a membrane filter of 0.22 μm pore size and concentrated using a concentration device (MILLIPORE, Microcon, 3 kDa). These samples correspond to the extracellular fraction.

Various volumes (10, 5, 2.5, 1 μL) of extracellular fractions from GLuc transformed cells and 10 μL of extracellular fraction from wild-type were separated by SDS-PAGE using a 12% polyacrylamide gel. The separated proteins were transferred onto nitrocellulose membrane and stained with Ponceau Red in order to control transfer efficiency. The nitrocellulose membrane was blocked overnight in milk 5% dissolved in TBS for immunodetection. Immunodetection was then performed using anti-GLuc (BIOLABS, E8023S) (1:2000 in TBS-T containing milk 1% for 2h at room temperature). Membranes were then washed with TBS-T (6 times, 5 minutes, room temperature). Binding of anti-GLuc antibody was revealed upon incubation with a secondary horseradish peroxidase-conjugated goat anti-rabbit IgG antibody (SIGMA-ALDRICH, A0545) diluted at 1:10,000 in TBS-T containing milk 1% for 1.5h at room temperature. Membranes were then washed with TBS-T (6 times, 5 minutes, room temperature) followed by a final wash with TBS (5 minutes, room temperature). Final development of the blots was performed by chemiluminescence method.

As depicted in figure 3, no signal was detected in the extracellular fraction from the wild-type cell line. A single band was detected in the extracellular fraction of the full-length GLuc cell line at approximately 18 kDa. It corresponds to the size predicted using a mass prediction software (http://expasy.org/tools/pi_tool.html) after the cleavage of the signal peptide. Indeed, this software predicts a molecular weight at 19.9 kDa for the full-length GLuc and 18.17 kDa for the protein after being cleaved. This result demonstrates the production and the secretion into the culture medium of the recombinant GLuc protein. It also proves the functionality of the native signal peptide from *Gaussia princeps* when expressed in *P. tricornutum.*

**Example 2 Secretion of murine erythropoietin in the culture medium of transformed *Phaeodactylum tricornutum***

A second experiment was carried out in *P. tricornutum* to test the functionality of exogenous signal peptide. *Phaeodactylum tricornutum* was transformed with a plasmid containing the murine erythropoietin coding sequence. This sequence encodes for a 192 amino acid precursor that contain a 26 amino acid signal peptide and a 166 amino acid mature protein containing 3 potential N-glycosylation sites.

a) Standard culture conditions of *Phaeodactylum tricornutum*

*Phaeodactylum tricornutum* strains used in this work were grown and prepared for genetic transformation as in example 1.a).

b) Expression constructs for EPO

The vector used for the expression construct of murine erythropoietin (EPOm) was the same vector used for the expression of luciferase in example 1.b). Murine erythropoietin is encoded by a 579 pb sequence (SEQ ID N°48).

The synthesis, digestion and insertion of EPOm sequence in the vector were prepared as the Luciferase sequence in example 1.b). Similarly, a vector bearing the EPOm coding sequence lacking the signal peptide was also realized (SEQ ID N°49).

c) Genetic transformation

The genetic transformation carried out in this experiment is described in the previous example 1.c).

d) Microalgae DNA extraction

DNA extraction carried out in this experiment is described in the previous example 1.d.

e) Polymerase chain reaction (PCR) analysis

The presence of the transgene was assessed by PCR as described in the previous example I.e. The sequence of primers used for the amplification EPOm transformed cells were 5'-CACGATGGGTTGTGCAGAAGG-3' (SEQ ID N° 50) and 5'-CGAAGCAGTGAAGTGAGGCTAC-3' (SEQ ID N° 51).

Results revealed a single band at 255bp for cells transformed with the constructs carrying the full-length EPOm or EPOm lacking its signal peptide (data not shown). No band was detected in cells transformed with the control vector. This result validates the incorporation of exogenous gene in the genome of *Phaeodactylum tricornutum.*

f) Erythropoïetin quantification

EPOm concentration was determined on the extracellular and intracellular fractions of wild-type and transformed cells of *P. tricornutum* using the ELISA (Enzyme-linked ImmunoSorbent Assay) method. An aliquote of the *P. tricornutum* culture at exponential phase of growth was collected and cells were separated from the culture medium by centrifugation (10 minutes, 2150g, 20°C). The supernatant was then filtered using a membrane filter of 0.22 $\mu$m pore size and corresponds to the extracellular fraction. The cell pellet was resuspended with a volume of fresh culture medium equivalent to the initial volume of the aliquote. The cellular suspension was then sonicated during 30 minutes at 4°C and centrifuged at 4500g during 5 minutes at 4°C. Supernatant was finally collected and corresponds to the intracellular fraction of *P. tricornutum.* EPOm quantification was realized on both fractions (intracellular and extracellular) using the ELISA Quantikine Mouse/Rat EPO Immunoassay Kit (R&D SYSTEMS), according to manufacturer's instructions. The lack of interference of the intracellular fraction with the ELISA detection was verified by the addition of a known quantity of recombinant murine EPO (R&D SYSTEMS) to this fraction.

EPOm was mainly detected in the extracellular fraction (0.52 mg/L) when compared to the intracellular fraction (0.02 mg/L) of cells transformed with full-length EPOm construct. Murine EPO could not be detected in both fractions from wild type cells transformed with EPOm construct lacking its signal peptide or wild-type cells. These results revealed that murine EPO was produced with most of the protein being secreted in the culture medium of transformed *P. tricornutum.* It demonstrates the functionality of a murine signal peptide when expressed in the diatom *P.tricornutum.*

g) Immunoblotting analysis

Aliquotes of wild-type and transformed cells of *P. tricornutum* culture at exponential phase of growth were collected and cells were separated from the culture medium by centrifugation (10 minutes, 2150g, 20°C). The supernatant was filtered using a membrane filter of 0.22 $\mu$m pore size and concentrated using a concentration device (MILLIPORE, Microcon, 3 kDa). These samples correspond to the extracellular fraction.

The immunodetection of EPO was performed on the extracellular fractions as in example 1.g) by using anti-EPO (R&D SYSTEMS, AF959) antibodies. Binding of said anti-EPO antibody was revealed upon incubation with a secondary horseradish peroxidase-conjugated rabbit anti-goat IgG (SIGMA-ALDRICH, A8919) in the same condition as in example 1.e).

As depicted in figure 4, no band was visible in the sample from the wild-type cell line. A single band was detected in the extracellular fraction purified from the transformed cells with a molecular weight around 25 kDa. As expected, a band at 34 kDa was detected for the commercial recombinant murine EPO used as control. Erythropoietin possesses 3 potential N-glycosylation sites. Since the predicted molecular weight of the amino acids backbone of EPO is 20 kDa, this result suggested that the protein was glycosylated. The difference of molecular weight between native murine EPO and EPO produced in *P. tricornutum* could originate from a difference in the glycan moieties. This result also strongly suggested that EPO followed the classical ER-golgi secretory pathway allowing the glycosylation of this protein.

Altogether, data from the ELISA and western blot experiments prove that EPO was produced and secreted in the culture medium of *P. tricornutum.* These results also demonstrate the functionality of the native signal peptide of the murine EPO.

**Example 3 Secretion of human interleukin-2 in the culture medium of transformed *Phaeodactylum tricornutum***

A third experiment is carried out in *P. tricornutum* to test the functionality of exogenous signal peptides. *Phaeodactylum tricornutum* is transformed with a plasmid containing the human interleukin-2 coding sequence. This sequence encodes for a 153 amino acid precursor that contain a 20 amino acid signal peptide and a 133 amino acid mature protein containing one potential O-glycosylation site.

a) Standard culture conditions of *Phaeodactylum tricornutum*

*Phaeodactylum tricornutum* strains use in this work were grown and prepared for genetic transformation as in example 1.a).

b) Expression constructs for IL-2

The vector used for the expression construct of human IL-2 (IL-2) is the same vector used for the expression of luciferase in example 1.b). Human interleukin-2 is encoded by a 462 pb sequence (SEQ ID N°4).

The synthesis, digestion and insertion of human IL-2 sequences in vectors are prepared as the Luciferase sequence in example 1.b). Similarly, a vector bearing the IL-2 coding sequence lacking the signal peptide is also realized (SEQ ID N°52). A vector lacking the IL-2 coding sequence is used as control.

c) Genetic transformation

The genetic transformation carried out in this experiment is described in the previous example 1.c).

d) Interleukin-2 quantification

IL-2 concentrations are determined on the extracellular and intracellular fractions of wild-type and *P. tricornutum* transformed by full-length IL-2 or IL-2 lacking its signal peptide. An aliquote of the *P. tricornutum* culture at exponential phase of growth is collected and processed to collect both extracellular and intracellular fractions as described in example 2.f). IL-2 quantification is realized using the ELISA Quantikine Human IL-2 Immunoassay Kit (R&D SYSTEMS), according to manufacturer's instructions.

e) Immunoblotting of the secreted IL-2

Aliquotes of wild-type and transformed cells of *P. tricornutum* culture at exponential phase of growth are collected and cells are separated from the culture medium by centrifugation (10 minutes, 2150g, 20°C). Supernatants are filtered using a membrane filter of 0.22 μm pore size and concentrated using a concentration device (MILLIPORE, Microcon, 3 kDa). These samples correspond to the extracellular fraction.

The immunodetection of IL-2 is performed on various volume of purified fractions (5, 10, 15 μL) by using anti-IL-2 (R&D SYSTEMS, AB-202-NA) antibodies. Binding of said anti-IL-2 antibody is revealed upon incubation with a secondary horseradish peroxidase-conjugated rabbit anti-goat IgG (SIGMA-ALDRICH, A8919) in the same condition as in example 1.e).

f) Purification of the secreted IL-2

The secreted IL-2 is purified by chromatography method. Culture medium of *P. tricornutum* at exponential phase of growth is collected and cells are separated from the culture medium by centrifugation (10 minutes, 2150g, 20°C). The supernatant is filtered using a membrane filter of 0.22 μm pore size, concentrated 10 times, and buffer-exchanged with 25 mM ammonium acetate, pH 5 using a concentration device (MILLIPORE, Amicon Ultra-15, 3 kDa).

Purification is performed using the AKTA FPLC system (GE Healthcare) and a CM Sepharose column (GE Healthcare). The column is equilibrated with 25 mM ammonium acetate, pH 5. The sample is then loaded to the column. The column is washed extensively, and bound IL-2 is eluted with a step gradient of 0-1 M sodium chloride in 25 mM ammonium acetate, pH 5. The peak is collected and loaded on a Sephadex G-50 column equilibrated with 5 mM sodium phosphate buffer, pH 7.4. The desalted protein is collected and concentrated using a concentration device (MILLIPORE, Amicon Ultra-15, 3 kDa). Concentration of IL-2 in collected fractions is determined by ELISA method and the purity of IL-2 is assessed by immunoblotting analysis.

g) Analysis of IL-2 protein sequence

Fifteen μL of IL-2 purified from the extracellular medium is separated by SDS-PAGE using a 12% polyacrylamide gel. Protein bands are stained with Coomassie brilliant blue CBB R-350 (Amersham Bioscience). The CBB-stained proteins on SDS-PAGE corresponding to IL-2 is excised and digested with sequencing grade modified trypsin (Promega). The gel piece is washed with 50% acetonitrile/0.1 M ammonium bicarbonate, and then dehydrated with acetonitrile. The protein in gel pieces is reduced with 10 mM dithiothreitol and alkylated with 55mM iodoacetamide. The gel piece is washed once with 20 mM ammonium bicarbonate and dehydrated with acetonitrile. The trypsin solution is added to the gel piece, and the enzyme reaction is allowed to proceed overnight at 37 °C. After digestion, the supernatant is acidified by adding trifluoroacetic acid and immediately subjected to mass spectrometry or stored in a freezer until analysis. Nano-LC/MS/MS experiments are performed on Q-TOF 2 and Ultima API hybrid mass spectrometers (Waters) equipped with a nano-electrospray ion source and a CapLC system (Waters). The mass spectrometers are operated in data-directed acquisition mode. For protein identification, all MS/MS spectra are searched using the SwissProt data-base.

**Example 4 Secretion of enhanced Green Fluorescent Protein (eGFP) in the culture medium of *Phaeodactylum tricornutum***

A fourth experiment is carried out to test the ability of the exogenous signal peptide from *Gaussia princeps* luciferase to drive the secretion of the naturally cytosolic eGFP. This chimeric sequence encodes for a 255 amino acids precursor that contains a 17 amino acids signal peptide from *Gaussia princeps* luciferase and a 238 amino acids mature protein.

a) Standard culture conditions of *Phaeodactylum tricornutum*

*Phaeodactylum tricornutum* strains use in this work were grown and prepared for genetic transformation as in example 1.a).

b) Expression constructs for the chimeric eGFP

The vector used for the expression construct of the chimeric eGFP is the same vector used for the expression of luciferase in example 1.b). The chimeric eGFP is encoded by a 768 pb sequence (SEQ ID N°53). Alternatively a 786 pb sequence containing a Histidine tag at the carboxyl-terminus of the protein is also realized (SEQ ID N°54).

The synthesis, digestion and insertion of both sequences in vectors are prepared as the Luciferase sequence in example 1.b). A vector lacking the chimeric GFP coding sequence is used as control.

c) Genetic transformation

The genetic transformation carried out in this experiment is described in the previous example 1.c).

d) Immunoblotting analysis

Aliquotes of wild-type and transformed cells of *P. tricornutum* culture at exponential phase of growth are collected and cells are separated from the culture medium by centrifugation (10 minutes, 2150g, 20°C). Supernatants are filtered using a membrane filter of 0.22 μm pore size and concentrated using a concentration device (MILLIPORE, Microcon, 3 kDa). These samples correspond to the extracellular fraction.

The immunodetection of the chimeric eGFP is performed on the extracellular fractions in the same condition as in example 1.e) except that a horseradish peroxidase-conjugated anti-GFP (Santa Cruz, sc-9996) antibody is used (1:2000 in TBS-T containing milk 1% for 2h at room temperature).

e) Purification of the secreted chimeric eGFP

The secreted chimeric eGFP fused to the histidine tag is purified by chromatography method. Culture medium of *P. tricornutum* at exponential phase of growth is collected and cells are separated from the culture medium by centrifugation (10 minutes, 2150g, 20°C). The supernatant is filtered using a membrane filter of 0.22 μm pore size, concentrated 10 times, and buffer-exchanged with 20 mM Tris, pH 9 containing 5 mM imidazole using a concentration device (MILLIPORE, Amicon Ultra-15, 3 kDa). Purification is performed using the AKTA FPLC system (GE Healthcare) and a Ni Sepharose column (GE Healthcare). The column is equilibrated with 20 mM Tris, pH 9.0 buffer containing 5 mM imidazole and the sample is then loaded. The column is washed with buffer containing 10 mM imidazole followed by elution with buffer containing 200 mM imidazole. The peak is collected and loaded on a Sephadex G-50 column equilibrated with 5 mM sodium phosphate buffer, pH 7.4. The desalted protein is collected and concentrated using a concentration device (MILLIPORE, Amicon Ultra-15, 3 kDa).

f) Analysis of the chimeric eGFP protein sequence

Fifteen μL of the purified chimeric eGFP is separated by SDS-PAGE using a 12% polyacrylamide gel. Protein bands are stained with Coomassie brilliant blue CBB R-350 (Amersham Bioscience). The CBB-stained proteins on SDS-PAGE corresponding to chimeric eGFP is excised and digested with sequencing grade modified trypsin (Promega) or arginine-C (Princeton Separations). The gel piece is washed with 50% acetonitrile/0.1 M ammonium bicarbonate, and then dehydrated with acetonitrile. The protein in gel pieces is reduced with 10 mM dithiothreitol and alkylated with 55mM iodoacetamide. The gel piece is washed once with 20 mM ammonium bicarbonate and dehydrated with acetonitrile. The trypsin solution is added to the gel piece, and the enzyme reaction is allowed to proceed overnight at 37 °C. Alternatively, the arginine-C solution is added to the gel piece, and the enzyme reaction is allowed to proceed overnight at room temperature. Both supernatants from trypsin or arginine-C are acidified by adding trifluoroacetic acid and immediately subjected to mass spectrometry or stored in a freezer until analysis. Nano-LC/MS/MS experiments are performed on Q-TOF 2 and Ultima API hybrid mass spectrometers (Waters) equipped with a nano-electrospray ion source and a CapLC system (Waters). The mass spectrometers are operated in data-directed acquisition mode. For protein identification, all MS/MS spectra are searched using the SwissProt data-base.

**Example 5 Expression of proteins of therapeutic interest as listed in Table I**

The term "PROTEIN" corresponds herein to the name of the protein of therapeutic interest to be secreted in the extracellular medium of diatoms, said name being listed in Table I, and derivatives thereof.

a) Standard culture conditions *of Phaeodactylum tricornutum*

Diatoms are grown and prepared for the genetic transformation as in example 1.a). The conditions of culture may be adapted to the species used for the secretion of PROTEIN.

b) Expression constructs for the protein of therapeutic interest

The vector used for the expression construct of PROTEIN is the same vector used in example 1.b). PROTEIN is encoded by the nucleic acid sequence listed in Table I. The synthesis, digestion and insertion of PROTEIN sequence in the vector are prepared as in example 1.b).

c) Genetic transformation

The transformation carried out on diatoms is described in the example 1.c).

d) Protein quantification

PROTEIN concentration is determined on the extracellular and intracellular fractions of transformed diatoms by using the ELISA method as described in example 2.f).

e) Immunoblotting analysis

The immunodetection of PROTEIN is performed as in example 1.g) by using anti-PROTEIN antibodies. Binding of said anti-PROTEIN antibodies is revealed upon incubation with a secondary antibody directed against anti-PROTEIN antibodies.

## Claims

1. A transformed diatom comprising a nucleic acid sequence operatively linked to a promoter, wherein said nucleic acid sequence encodes an amino acid sequence comprising :

   (i) an heterologous signal peptide; and
   (ii) a polypeptide,
   said heterologous signal peptide leading to the secretion of said polypeptide in the extracellular medium of said transformed diatom.

2. The transformed diatom according to claim 1, wherein said diatom is selected from the group comprising Phaeodactylacaeae diatoms.

3. The transformed diatom according to any one of claims 1 or 2, wherein said diatom is *Phaeodactylum tricornutum.*

4. The transformed diatom according to any one of claims 1 to 3, wherein
   said polypeptide is a heterologous polypeptide, and
   said heretologous signal peptide is the signal peptide of said heterologous polypeptide, said signal peptide leading to the secretion of said polypeptide in the extracellular medium of the organism of said polypeptide.

5. The transformed diatom according to any one of claims 1 to 4, wherein the polypeptide is an animal polypeptide of animal origin, preferably a mammalian polypeptide of mammalian origin and most preferably a human polypeptide of human origin.

6. The transformed diatom according to any one of claims 1 to 5, wherein said polypeptide is selected from the group comprising erythropoietin, cytokines such as interferons, antibodies and their fragments, coagulation factors, hormones, beta-glucocerebrosidase, pentraxin-3, anti-TNFs, α-glucosidase acide, α-L-iduronidase and derivatives thereof .

7. The transformed diatom according to any one of claims 1 to 6, wherein said nucleic acid sequence is selected from the group comprising the nucleic acid sequences as listed in Table I and derivatives thereof.

8. A method for producing a polypeptide which is secreted in the extracellular medium, said method comprising the steps of:

   (i) culturing a transformed diatom as defined in any one of claims 1 to 5;
   (ii) harvesting the extracellular medium of said culture; and
   (iii) purifying the secreted polypeptide in said extracellular medium.

9. The method according to claim 7, wherein said method comprises a step (iv) of determining the glycosylation pattern of said polypeptide.

10. The method according to any one of claims 7 or 8, wherein said method leads to the secretion in the extracellular medium of at least 25%, 50%, 75% or 90% of the polypeptide expressed in said diatom.

11. A use of a transformed diatom as defined in any one of claims 1 to 7 for the secretion of a polypeptide in the extracellular medium.

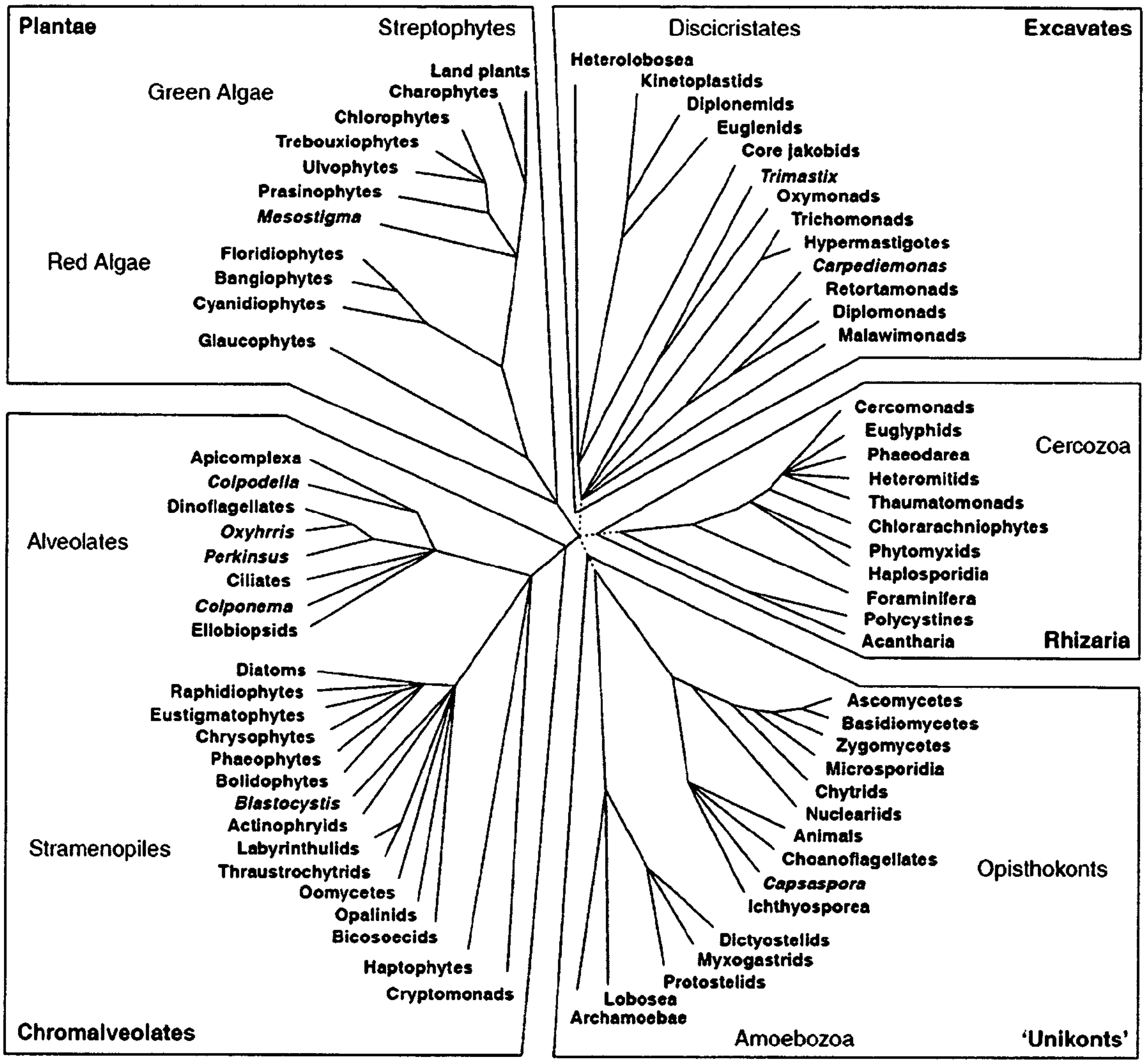
Plantae
Streptophytes
Green Algae
Land plants
Charophytes
Chlorophytes
Trebouxiophytes
Ulvophytes
Prasinophytes
Mesostigma
Red Algae
Floridiophytes
Bangiophytes
Cyanidiophytes
Glaucophytes
Discicristates
Excavates
Heterolobosea
Kinetoplastids
Diplonemids
Euglenids
Core jakobids
Trimastix
Oxymonads
Trichomonads
Hypermastigotes
Carpediemonas
Retortamonads
Diplomonads
Malawimonads
Cercomonads
Euglyphids
Phaeodarea
Cercozoa
Heteromitids
Thaumatomonads
Chlorarachniophytes
Phytomyxids
Haplosporidia
Foraminifera
Polycystines
Acantharia
Rhizaria
Alveolates
Apicomplexa
Colpodella
Dinoflagellates
Oxyhrris
Perkinsus
Ciliates
Colponema
Ellobiopsids
Stramenopiles
Diatoms
Raphidiophytes
Eustigmatophytes
Chrysophytes
Phaeophytes
Bolidophytes
Blastocystis
Actinophryids
Labyrinthulids
Thraustrochytrids
Oomycetes
Opalinids
Bicosoecids
Haptophytes
Cryptomonads
Chromalveolates
Ascomycetes
Basidiomycetes
Zygomycetes
Microsporidia
Chytrids
Nucleariids
Animals
Choanoflagellates
Opisthokonts
Capsaspora
Ichthyosporea
Dictyostelids
Myxogastrids
Protostelids
Lobosea
Archamoebae
Amoebozoa
'Unikonts'

Figure 1

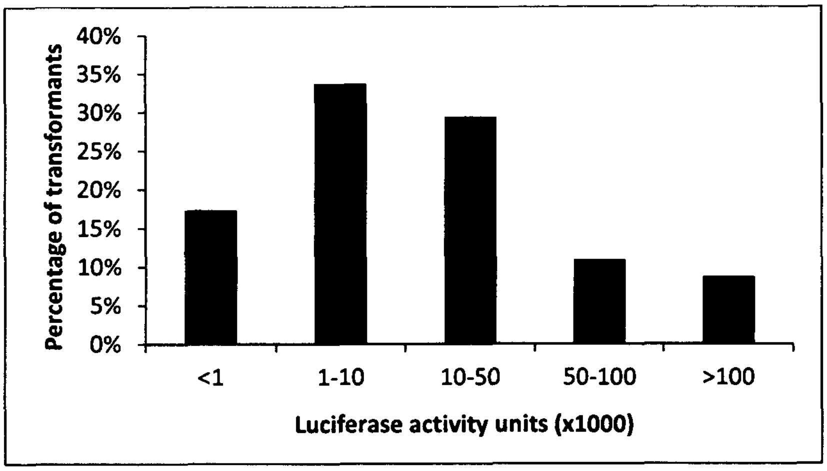


Figure 2

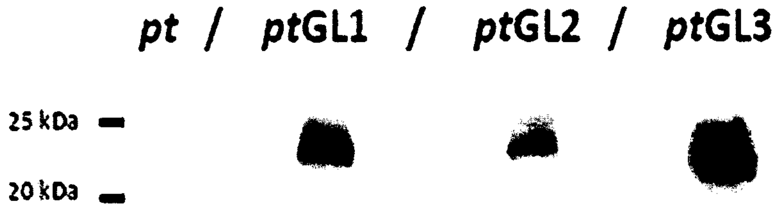


Figure 3

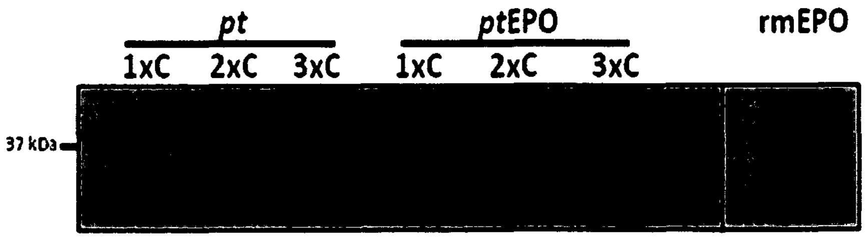


Figure 4

Europäisches Patentamt
European Patent Office
Office européen des brevets

# EUROPEAN SEARCH REPORT

Application Number
EP 10 01 3808

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/101160 A1 (IFREMER [FR]; CENTRE NAT RECH SCIENT [FR]; UNIV ROUEN [FR]; CADORET JE) 20 August 2009 (2009-08-20)<br>* page 49 *<br>----- | 1-11 | INV.<br>C12N15/82<br>C12P21/02<br><br>ADD.<br>C07K14/505<br>C07K14/55 |
| X | Anonymous: "Specialized Protein Expression Systems - Day 2 Agenda", Conference Specialized Protein Expression Systems, Cambridge, MA<br>,<br>2009, pages 1-3, XP002628989,<br>Retrieved from the Internet:<br>URL:http://www.healthtech.com/Conferences_Archive.aspx?id=89798<br>[retrieved on 2011-03-18]<br>* the whole document *<br>----- | 1-11 | |
| A,D | ZASLAVSKAIA LIOUDMILA A ET AL: "Transformation of the diatom Phaeodactylum tricornutum (Bacillariophyceae) with a variety of selectable marker and reporter genes. Correction of journal title", JOURNAL OF PHYCOLOGY,<br>vol. 36, no. 2, April 2000 (2000-04),<br>pages 379-386, XP002628990,<br>ISSN: 0022-3646<br>* the whole document *<br>----- | 1-11 | TECHNICAL FIELDS SEARCHED (IPC)<br><br>C12N<br>C07K<br>C12P |

The present search report has been drawn up for all claims

| Place of search | Date of completion of the search | Examiner |
|---|---|---|
| The Hague | 18 March 2011 | Lejeune, Robert |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 01 3808

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2011

| Patent document cited in search report | Publication date | Patent family member(s) | Publication date |
|---|---|---|---|
| WO 2009101160 A1 | 20-08-2009 | AU 2009214077 A1 | 20-08-2009 |
| | | CA 2715107 A1 | 20-08-2009 |
| | | EP 2090648 A1 | 19-08-2009 |
| | | EP 2257618 A1 | 08-12-2010 |
| | | US 2011045533 A1 | 24-02-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009101160 A


### Non-patent literature cited in the description


- **ZAVLASKAÏA et al.** Transformation of the diatom Phaeodactylum tricornutum (Bacillariophyceae) with a variety of selectable marker and reporter genes. *J Phycol.,* vol. 36, 379-386
- **VON HEIJNE G.** The signal Peptide. *J Membr Biol,* 1990, vol. 115, 195-201
- **EMANUELSSON O. et al.** Locating proteins in the cell using TargetP, SignalP and related tools. *Nat Protoc,* 2007, vol. 2, 953-971
- **VERNET K. ; SCHATZ G.** Protein translocation across membranes. *Science,* 1988, vol. 241, 1307-1313
- **SÉVENO et al.** Plant N-glycan profiling of minute amounts of material. *Anal. Biochem.,* 2008, vol. 379 (1), 66-72
- **STADLMANN et al.** Analysis of immunoglobulin glycosylation by LC-ESI-MS of glycopeptides and oligosaccharides. *Proteomics,* 2008, vol. 8, 2858-2871
- **THOMAS JL. et al.** A helium burst biolistic device adapted to penetrate fragile insect tissues. *Journal of Insect Science,* 2001, 1-9